# EUROPEAN PATENT SPECIFICATION

(11) **EP 0 690 867 B1**
(45) Date of publication and mention of the grant of the patent: **19.02.2003**
(21) Application number: 94911675.0
(22) Date of filing: 21.03.1994
(51) Int. Cl.: C07F 7/08, C07D 305/14, A61K 31/335, C07D 409/12, C07D 407/12

(54) **TAXANES HAVING FURYL OR THIENYL SUBSTITUTED SIDE-CHAIN**
TAXANE MIT FURYL-ODER THIENYL SUBSTITUIERTEN SEITENKETTEN
TAXANES A CHAINE LATERALE SUBSTITUEE PAR FURYLE OU THIENYLE

(30) Priority: 22.03.1993 US 34852; 20.07.1993 US 94717
(43) Date of publication of application: 10.01.1996
(60) Divisional application: 02009839.8
(73) Proprietor: FLORIDA STATE UNIVERSITY, Tallahassee, Florida 32310 (US)
(72) Inventor: HOLTON, Robert, A., 2035 East Paul Dirac Drive, Tallahassee, FL 32310 (US); NADIZADEH, Hossain, 2035 East Paul Dirac Drive, Tallahassee, FL 32310 (US); BIEDIGER, Ronald, J., 2035 East Paul Dirac Drive, Tallahassee, FL 32310 (US); RENGAN, Kasthuri, 83-45 Vietor Avenue, New York 11373 (US); SUZUKI, Yukio, 2035 East Paul Dirac Drive, Tallahassee, FL 32310 (US); TAO, Chunlin, 2035 East Paul Dirac Drive, Tallahassee, FL 32310 (US); CHAI, Ki-byung, 2035 East Paul Dirac Drive, Tallahassee, FL 32310 (US); IDMOUMAZ, Hamid, 2035 East Paul Dirac Drive, Tallahassee, FL 32310 (US)
(74) Representative: Eyles, Christopher Thomas
(86) International application number: US9403097
(87) International publication number: WO94021651

(56) References cited:
- US-A- 4 942 184
- US-A- 5 175 315
- US-A- 5 227 400

## Description

### BACKGROUND OF THE INVENTION

The present invention is directed to novel taxanes which have utility as antileukemia and antitumor agents.

The taxane family of terpenes, of which taxol is a member, has attracted considerable interest in both the biological and chemical arts. Taxol is a promising cancer chemotherapeutic agent with a broad spectrum of antileukemic and tumor-inhibiting activity. Taxol has a 2'R, 3'S configuration and the following structural formula: 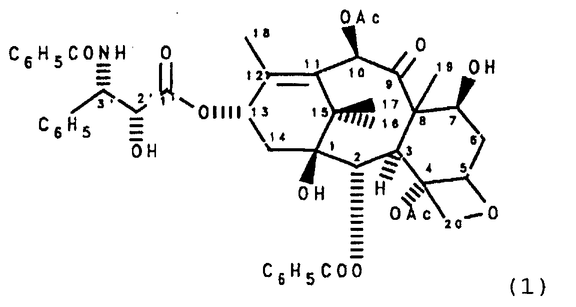 wherein Ac is acetyl. Because of this promising activity, taxol is currently undergoing clinical trials in both France and the United States.

Colin et al. reported in U.S. Patent No. 4,814,470 that taxol derivatives having structural formula **(2)** below, have an activity significantly greater than that of taxol **(1)**. 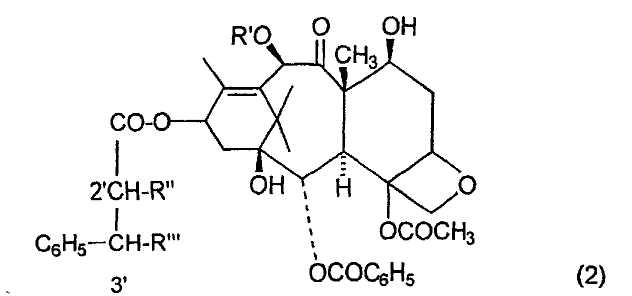 R' represents hydrogen or acetyl and one of R" and R"' represents hydroxy and the other represents tert-butoxycarbonylamino and their stereoisomeric forms, and mixtures thereof. The compound of formula (2) in which R' is hydrogen, R" is hydroxy, and R"' is tert-butoxycarbonylamino having the 2'R, 3'S configuration is commonly referred to as taxotere.

Haugwitz et al. describe in U.S. Patent No. 4,942,184 certain water soluble antineoplastic derivatives of taxol which have been acylated in the 2'-position.

In U.S. Patent No. 5,175,315 Holton describes a semisynthetic approach for the preparation of taxol and other taxanes using a reaction between certain β-lactams and alcohols.

Although taxol and taxotere are promising chemotherapeutic agents, they are not universally effective. Accordingly, a need remains for additional chemotherapeutic agents.

### SUMMARY OF THE INVENTION

Among the objects of the present invention, therefore, is the provision of novel taxane derivatives which are valuable antileukemia and antitumor agents.

Briefly, therefore, the present invention is directed to taxane derivatives having a C13 side chain which includes a furyl or thienyl substituent. In a preferred embodiment, the taxane derivative has a tricyclic or tetracyclic core and corresponds to the formula: 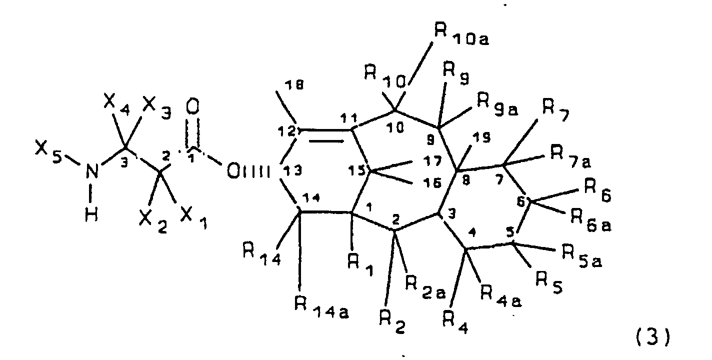 wherein
X₁ is -OX₆, -SX₇, or -NX₈X₉;
X₂ is hydrogen, alkyl, alkenyl, alkynyl, aryl, or heteroaryl;
X₃ and X₄ are independently hydrogen, alkyl, alkenyl, alkynyl, aryl, heteroaryl, acyl or heterosubstituted alkyl, alkenyl, alkynyl, aryl or heteroaryl, provided, however, that X₃ and X₄ are not both acyl;
X₅ is -COX₁₀, -COOX₁₀, -COSX₁₀, -CONX₈X₁₀, or -SO₂X₁₁;
X₆ is hydrogen, alkyl, alkenyl, alkynyl, aryl, heteroaryl, or a hydroxy protecting group;
X₇ is alkyl, alkenyl, alkynyl, aryl, heteroaryl, or sulfhydryl protecting group;
X₈ is hydrogen, alkyl, alkenyl, alkynyl, aryl, heteroaryl, or heterosubstituted alkyl, alkenyl, alkynyl, aryl or heteroaryl;
X₉ is an amino protecting group;
X₁₀ is alkyl, alkenyl, alkynyl, aryl, heteroaryl, or heterosubstituted alkyl, alkenyl alkynyl, aryl or heteroaryl;
X₁₁ is alkyl, alkenyl, alkynyl, aryl, heteroaryl, -OX₁₀, or -NX₈X₁₄;
X₁₄ is hydrogen, alkyl, alkenyl, alkynyl, aryl, or heteroaryl;
R₁₄ is hydrogen, alkyl, alkenyl, alkynyl, aryl, or heteroaryl, hydroxy, protected hydroxy or together with R₁ forms a carbonate;
R₁₄ₐ is hydrogen, alkyl, alkenyl, alkynyl, aryl, or heteroaryl;
R₁₀ is hydrogen or together with R₁₀ₐ forms an oxo;
R₁₀ₐ is hydrogen, -OCOR₂₉, hydroxy, or protected hydroxy, or together with R₁₀ forms an oxo;
R₉ is hydrogen or together with R₉ₐ forms an oxo;
R₉ₐ is hydrogen, hydroxy, protected hydroxy, acyloxy, or together with R₉ forms an oxo;
R₇ₐ is hydrogen or together with R₇ forms an oxo;
R₇ is hydroxy, or together with R₇ₐ forms an oxo;
R₆ is hydrogen, alkyl, alkenyl, alkynyl, aryl, or heteroaryl, hydroxy, protected hydroxy or together with R₆ₐ forms an oxo;
R₆ₐ is hydrogen, alkyl, alkenyl, alkynyl, aryl, or heteroaryl, hydroxy, protected hydroxy or together with R₆ forms an oxo;
R₅ is hydrogen or together with R₅ₐ forms an oxo;
R₅ₐ is hydrogen, hydroxy, protected hydroxy, acyloxy, together with R₅ forms an oxo, or together with R₄ and the carbon atoms to which they are attached form an oxetane ring;
R₄ is hydrogen, together with R₄ₐ forms an oxo, or together with R₅ₐ and the carbon atoms to which they are attached form an oxetane ring;
R₄ₐ is hydrogen, alkyl, alkenyl, alkynyl, aryl, heteroaryl, cyano, hydroxy, -OCOR₃₀, or together with R₄ forms an oxo, oxirane or methylene;
R₂ is hydrogen, hydroxy, or -OCOR₃₁;
R₂ₐ is hydrogen or taken together with R₂ forms an oxo;
R₁ is hydrogen, hydroxy, protected hydroxy; and
R₂₉, R₃₀, and R₃₁ are independently hydrogen, alkyl, alkenyl, alkynyl, monocyclic aryl or monocyclic heteroaryl;
provided, however, that at least one of X₃, X₄ and X₁₀ is furyl or thienyl; and either (i) R₁₀ₐ is hydrogen or together with R₁₀ forms an oxo; (ii) R₉ₐ is hydrogen, hydroxy, protected hydroxy, or acyloxy; (iii) R₇ together with R₇ₐ forms an oxo; (iv) R₄ₐ is hydrogen, alkyl, alkenyl, alkynyl, aryl, heteroaryl, cyano, hydroxy, -OCOR₃₀ wherein R₃₀ is other than methyl, or together with R₄ forms an oxo, oxirane, or methylene; (v) R₂ is hydrogen, hydroxy, or -OCOR₃₁ wherein R₃₁ is other than phenyl; (vi) R₁ is other than hydroxy; (vii) R₁₄ is other than hydrogen.

Other objects and features of this invention will be in part apparent and in part pointed out hereinafter.

### DETAILED DESCRIPTION OF THE PREFERRED EMBODIMENTS

As used herein "Ar" means aryl; "Ph" means phenyl; "Ac" means acetyl; "Et" means ethyl; "R" means alkyl unless otherwise defined; "Bu" means butyl; "Pr" means propyl; "TES" means triethylsilyl; "TMS" means trimethylsilyl; "TPAP" means tetrapropylammonium perruthenate; "DMAP" means p-dimethylamino pyridine; "DMF" means dimethylformamide; "LDA" means lithium diisopropylamide; "LAH" means lithium aluminum hydride; "Red-Al" means sodium bis (2-methoxyethoxy) aluminum hydride; "FAR" means 2-chloro-1,1,2-trifluorotriethylamine; "AIBN" means azo-(bis)-isobutyronitrile; "10-DAB" means 10-desacetylbaccatin III; "protected hydroxy" means -OR wherein R is a hydroxy protecting group; "sulfhydryl protecting group" includes, but is not limited to, hemithioacetals such as 1-ethoxyethyl and methoxymethyl, thioesters, or thiocarbonates; "amine protecting group" includes, but is not limited to, carbamates, for example, 2,2,2-trichloroethylcarbamate or tertbutyl-carbamate; and "hydroxy protecting group" includes, but is not limited to, ethers such as methyl, t-butyl, benzyl, p-methoxybenzyl, p-nitrobenzyl, allyl, trityl, methoxymethyl, methoxyethoxymethyl, ethoxyethyl, tetrahydropyranyl, tetrahydrothiopyranyl, and trialkylsilyl ethers such as trimethylsilyl ether, triethylsilyl ether, dimethylarylsilyl ether, triisopropylsilyl ether and t-butyldimethylsilyl ether; esters such as benzoyl, acetyl, phenylacetyl, formyl, mono-, di-, and trihaloacetyl such as chloroacetyl, dichloroacetyl, trichloroacetyl, trifluoroacetyl; and carbonates including but not limited to alkyl carbonates having from one to six carbon atoms such as methyl, ethyl, n-propyl, isopropyl, n-butyl, t-butyl, isobutyl, and n-pentyl; alkyl carbonates having from one to six carbon atoms and substituted with one or more halogen atoms such as 2,2,2-trichloroethoxymethyl and 2,2,2-trichloro-ethyl; alkenyl carbonates having from two to six carbon atoms such as vinyl and allyl; cycloalkyl carbonates having from three to six carbon atoms such as cyclopropyl, cyclobutyl, cyclopentyl and cyclohexyl; and phenyl or benzyl carbonates optionally substituted on the ring with one or more C₁₋₆ alkoxy, or nitro. Other hydroxyl, sulfhydryl and amine protecting groups may be found in "Protective Groups in Organic Synthesis" by T. W. Greene, John Wiley and Sons, 1981.

The alkyl groups described herein, either alone or with the various substituents defined hereinabove are preferably lower alkyl containing from one to six carbon atoms in the principal chain and up to 15 carbon atoms. They may be straight or branched chain and include methyl, ethyl, propyl, isopropyl, butyl, hexyl and the like.

The alkenyl groups described herein, either alone or with the various substituents defined hereinabove are preferably lower alkenyl containing from two to six carbon atoms in the principal chain and up to 15 carbon atoms. They may be straight or branched chain and include ethenyl, propenyl, isopropenyl, butenyl, isobutenyl, hexenyl, and the like.

The alkynyl groups described herein, either alone or with the various substituents defined hereinabove are preferably lower alkynyl containing from two to six carbon atoms in the principal chain and up to 15 carbon atoms. They may be straight or branched chain and include ethynyl, propynyl, butynyl, isobutynyl, hexynyl, and the like.

The aryl moieties described herein, either alone or with various substituents, contain from 6 to 15 carbon atoms and include phenyl. Substituents include alkanoxy, protected hydroxy, halogen, alkyl. aryl, alkenyl, acyl, acyloxy, nitro, amino, and amido. Phenyl is the more preferred aryl.

The heteroaryl moieties described herein, either alone or with various substituents, contain from 5 to 15 atoms and include, furyl, thienyl, pyridyl and the like. Substituents include alkanoxy, protected hydroxy, halogen, alkyl, aryl, alkenyl, acyl, acyloxy, nitro, amino, and amido.

The acyloxy groups described herein contain alkyl, alkenyl, alkynyl, aryl or heteroaryl groups.

The substituents of the substituted alkyl, alkenyl, alkynyl, aryl, and heteroaryl groups and moieties described herein, may be alkyl, alkenyl, alkynyl, aryl, heteroaryl and/or may contain nitrogen, oxygen, sulfur, halogens and include, for example, lower alkoxy such as methoxy, ethoxy, butoxy, halogen such as chloro or fluoro, nitro, amino, and keto.

In accordance with the present invention, it has been discovered that compounds corresponding to structural formula **3** show remarkable properties, in vitro , and are valuable antileukemia and antitumor agents. Their biological activity has been determined in vitro, using tubulin assays according to the method of Parness et al., J. Cell Biology, 91: 479-487 (1981) and human cancer cell lines, and is comparable to that exhibited by taxol and taxotere.

Preferably, X₁ is -OH, X₂ is hydrogen,, X₃ is furyl or thienyl, X₄ is hydrogen, X₅ is -COX₁₀ or -COOX₁₀, and X₁₀ is alkyl, alkenyl, alkynyl, aryl, furyl, thienyl or other heteroaryl and the taxane has the 2'R, 3'S configuration. In a particularly preferred embodiment, X₃ is furyl or thienyl, X₄ is hydrogen, and either X₅ is -COX₁₀ wherein X₁₀ is selected from furyl, thienyl, alkyl substituted furyl or thienyl, tert-, iso- or n-butyl, ethyl, iso- or n-propyl, cyclohexyl, allyl, crotyl, 1,3-diethoxy-2-propyl, 2-methoxyethyl, neopentyl, and PhCH₂-, or X₅ is -CONX₈X₁₀ wherein X₈ and X₁₀ are both phenyl or X₈ is hydrogen and X₁₀ is n-propyl, phenyl or ethyl.

In preferred embodiments of the present invention, the taxane has a structure which differs from that of taxol or taxotere with respect to the C13 side chain and at least one other substituent. For example, R₂ may be hydroxy or -COR₃₁ wherein R₃₁ is hydrogen, alkyl or selected from the group comprising 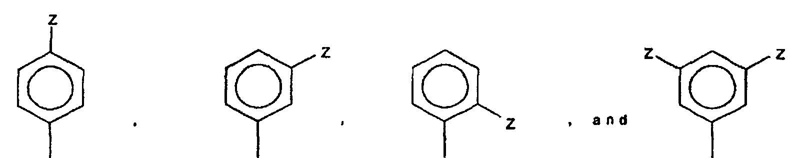 and Z is alkyl, hydroxy, alkoxy, halogen, or trifluoro methyl. R₉ may be hydrogen and R₉ₐ may be hydrogen or hydroxy, R₁₀ may be hydrogen and R₁₀ₐ may be acetoxy or other acyloxy or R₁₀ and R₁₀ₐ may be oxo, X₃ may be selected from isobutenyl, isopropyl, cyclopropyl, n-butyl, t-butyl, cyclobutyl, amyl, cyclohexyl, furyl, thienyl, pyridyl or the substituted derivatives thereof, and either X₅ is -COX₁₀ wherein X₁₀ is selected from furyl, thienyl, alkyl substituted furyl or thienyl, pyridyl, tert-, iso- or n-butyl, ethyl, iso- or n-propyl, cyclopropyl, cyclohexyl, allyl, crotyl, 1,3-diethoxy-2-propyl, 2-methoxyethyl, amyl, neopentyl, and PhCH₂-, or X₅ is -CONX₈X₁₀ wherein X₈ and X₁₀ are both phenyl or X₈ is hydrogen and X₁₀ is n-propyl, phenyl or ethyl.

Taxanes having the general formula **3** may be obtained by reacting a β-lactam with metal alkoxides having the taxane tricyclic or tetracyclic nucleus and a C-13 metallic oxide substituent to form compounds having a β-amido ester substituent at C-13. The β-lactams have the following structural formula: 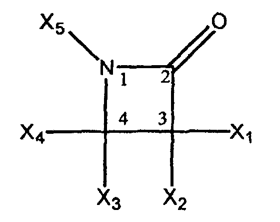 wherein X₁ - X₅ are as previously above.

The β-lactams can be prepared from readily available materials, as is illustrated in schemes A and B below: 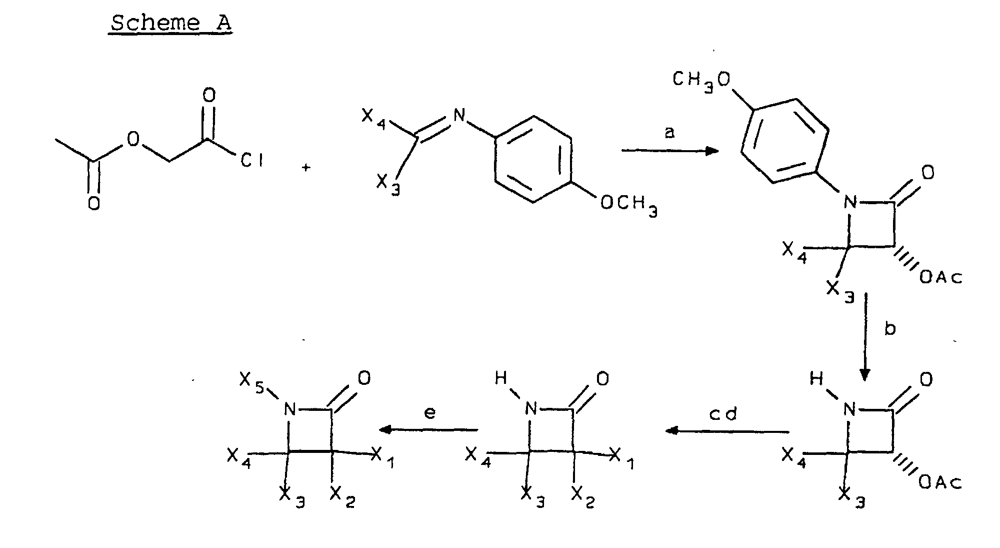 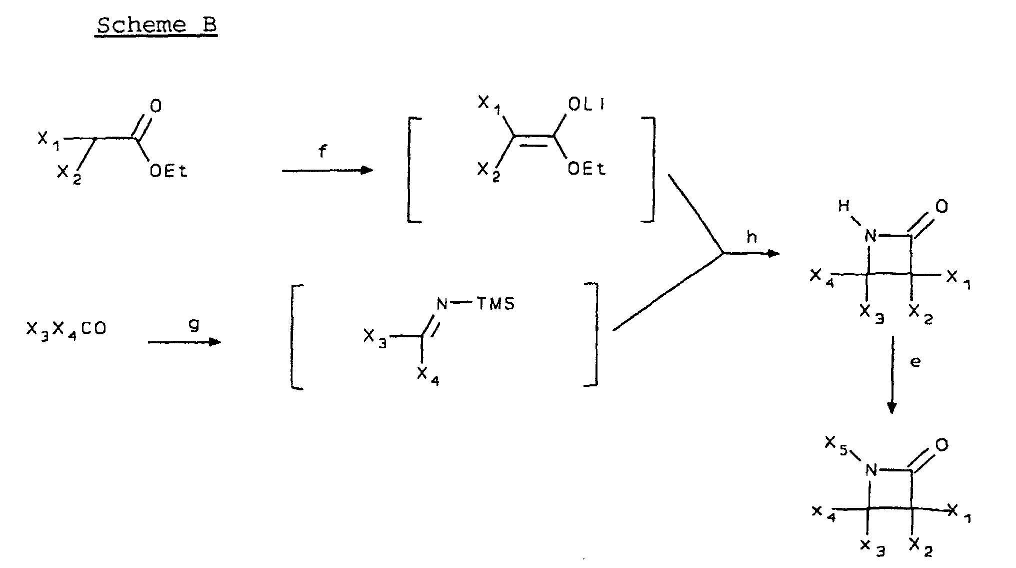 reagents: (a) triethylamine, CH₂Cl₂, 25°C, 18h; (b) 4 equiv ceric ammonium nitrate, CH₃CN, -10°C, 10 min; (c) KOH, THF, H₂O, O°C, 30 min, or pyrolidine, pyridine, 25 °C, 3h, (d) TESCl, pyridine, 25 °C, 30 min or 2-methoxypropene toluene sulfonic acid (cat.), THF, O°C, 2h; (e) n-butyllithium, THF, -78 °C, 30 min; and an acyl chloride or chloroformate (X₅ = -COX₁₀), sulfonyl chloride (X₅ = -COSX₁₀) or isocyanate (X₅ = -CONX₈X₁₀) ; (f) lithium diisopropyl amide, THF -78°C to -50°C; (g) lithium hexamethyldisilazide, THF -78°C to 0°C; (h) THF, -78°C to 25°C, 12h.

The starting materials are readily available. In scheme A, α-acetoxy acetyl chloride is prepared from glycolic acid, and, in the presence of a tertiary amine, it cyclocondenses with imines prepared from aldehydes and p-methoxyaniline to give 1-p-methoxyphenyl-3-acyloxy-4-arylazetidin-2-ones. The p-methoxyphenyl group can be readily removed through oxidation with ceric ammonium nitrate, and the acyloxy group can be hydrolyzed under standard conditions familiar to those experienced in the art to provide 3-hydroxy-4-arylazetidin-2-ones. In Scheme B, ethyl-α-triethylsilyloxyacetate is readily prepared from glycolic acid.

In Schemes A and B, X₁ is preferably -OX₆ and X₆ is a hydroxy protecting group. Protecting groups such as 2-methoxypropyl ("MOP"), 1-ethoxyethyl ("EE") are preferred, but a variety of other standard protecting groups such as the triethylsilyl group or other trialkyl (or aryl) silyl groups may be used. As noted above, additional hydroxy protecting groups and the synthesis thereof may be found in "Protective groups in Organic Synthesis" by T.W. Greene, John Wiley & Sons, 1981.

The racemic β-lactams may be resolved into the pure enantiomers prior to protection by recrystallization of the corresponding 2-methoxy-2-(trifluoromethyl) phenylacetic esters. However, the reaction described hereinbelow in which the β-amido ester side chain is attached has the advantage of being highly diastereoselective, thus permitting the use of a racemic mixture of side chain precursor.

The alkoxides having the tricyclic or tetracyclic taxane nucleus and a C-13 metallic oxide or ammonium oxide substituent have the following structural formula: 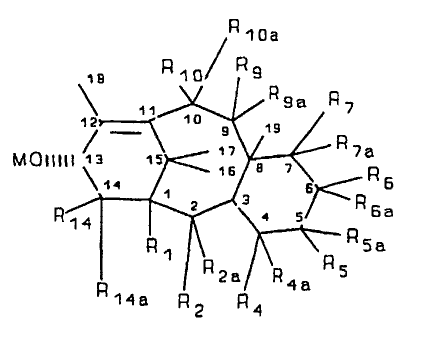 wherein R₁ - R₁₄ₐ are as previously defined and M comprises ammonium or is a metal optionally selected from the group comprising Group IA, Group IIA and transition metals, and preferably, Li, Mg, Na, K or Ti. Most preferably, the alkoxide has the tetracyclic taxane nucleus and corresponds to the structural formula: 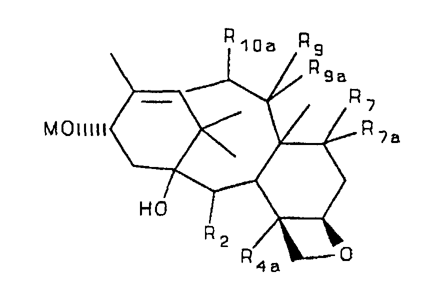 wherein M, R₂, R₄ₐ, R₇, R₇ₐ, R₉, R₉ₐ, R₁₀, and R₁₀ₐ are as previously defined.

The alkoxides can be prepared by reacting an alcohol having the taxane nucleus and a C-13 hydroxyl group with an organometallic compound in a suitable solvent. Most preferably, the alcohol is a protected baccatin III, in particular, 7-0-triethylsilyl baccatin III (which can be obtained as described by Greene, et al. in JACS 110: 5917 (1988) or by other routes) or 7,10-bis-O-triethylsilyl baccatin III.

As reported in Greene et al., 10-deacetyl baccatin III is converted to 7-O-triethylsilyl-10-deacetyl baccatin III according to the following reaction scheme: 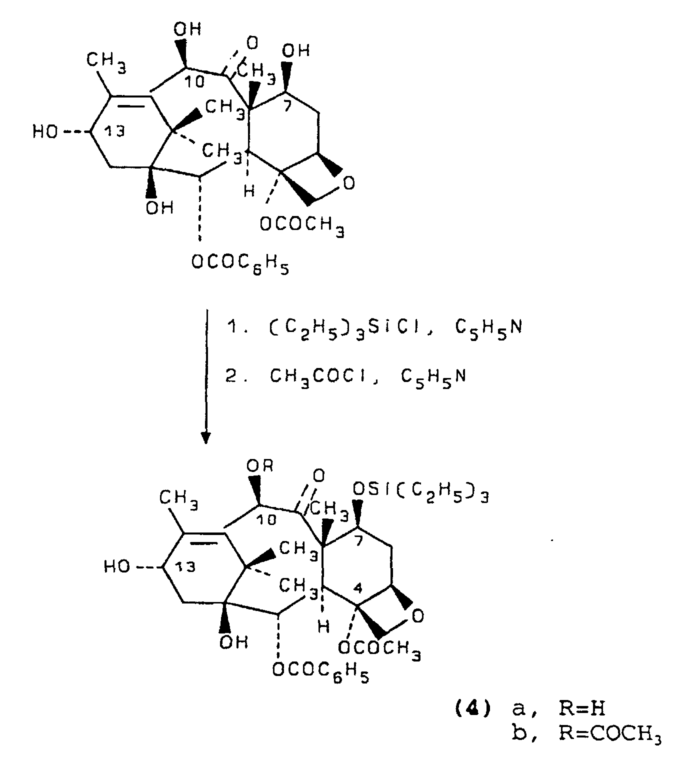 Under what is reported to be carefully optimized conditions, 10-deacetyl baccatin III is reacted with 20 equivalents of (C₂H₅)₃SiCl at 23°C under an argon atmosphere for 20 hours in the presence of 50 ml of pyridine/mmol of 10-deacetyl baccatin III to provide 7-triethylsilyl-10-deacetyl baccatin III **(4a)** as a reaction product in 84-86% yield after purification. The reaction product may then optionally be acetylated with 5 equivalents of CH₃COCl and 25 mL of pyridine/mmol of **4a** at 0 °C under an argon atmosphere for 48 hours to provide 86% yield of 7-O-triethylsilyl baccatin III **(4b).** Greene, et al. in JACS 110, 5917 at 5918 (1988).

The 7-protected baccatin III **(4b)** is reacted with an organometallic compound such as LHMDS in a solvent such as tetrahydrofuran (THF), to form the metal alkoxide 13-O-lithium-7-O-triethylsilyl baccatin III as shown in the following reaction scheme:

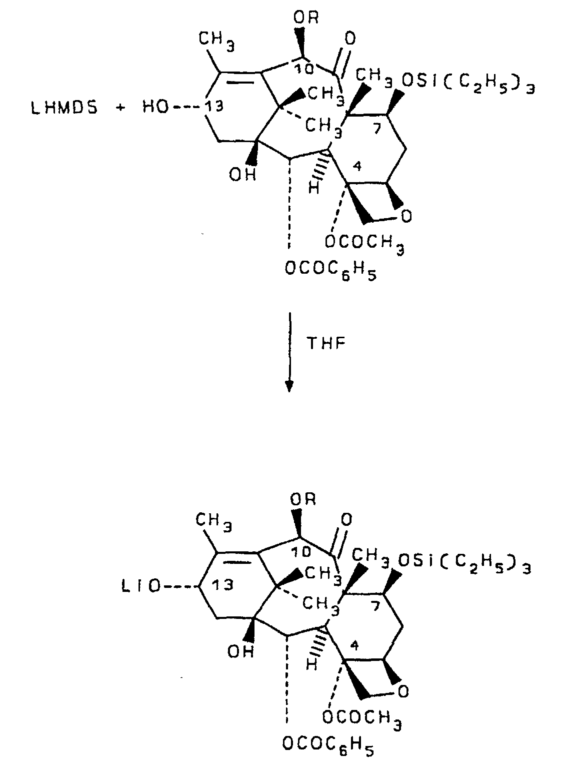

As shown in the following reaction scheme, 13-O-lithium-7-O-triethylsilyl baccatin III reacts with a β-lactam in which X₁ is preferably -OX₆, (X₆ being a hydroxy protecting group) and X₂ - X₅ are as previously defined to provide an intermediate in which the C-7 and C-2' hydroxyl groups are protected. The protecting groups are then hydrolyzed under mild conditions so as not to disturb the ester linkage or the taxane substituents.

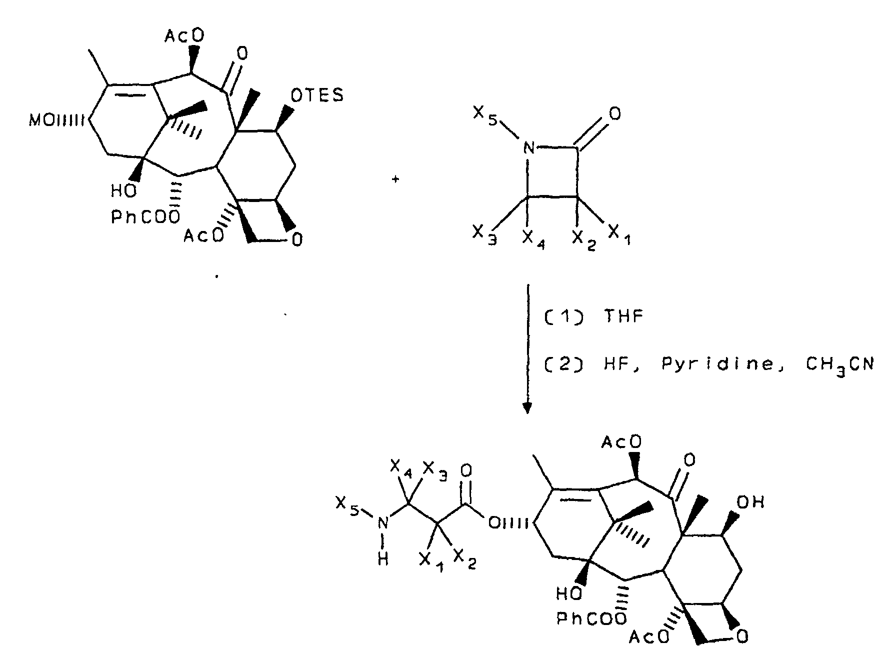

Both the conversion of the alcohol to the alkoxide and the ultimate synthesis of the taxane derivative can take place in the same reaction vessel. Preferably, the β-lactam is added to the reaction vessel after formation therein of the alkoxide.

Compounds of formula **3** of the instant invention are useful for inhibiting tumor growth in animals including humans and are preferably administered in the form of a pharmaceutical composition comprising an effective antitumor amount of compound of the instant invention in combination with a pharmaceutically acceptable carrier or diluent.

Antitumor compositions herein may be made up in any suitable form appropriate for desired use; e.g., oral, parenteral or topical administration. Examples of parenteral administration are intramuscular, intravenous, intraperitoneal, rectal and subcutaneous administration.

The diluent or carrier ingredients should not be such as to diminish the therapeutic effects of the antitumor compounds.

Suitable dosage forms for oral use include tablets, dispersible powders, granules, capsules, suspensions, syrups, and elixirs. Inert diluents and carriers for tablets include, for example, calcium carbonate, sodium carbonate, lactose and talc. Tablets may also contain granulating and disintegrating agents such as starch and alginic acid, binding agents such as starch, gelatin and acacia, and lubricating agents such as magnesium stearate, stearic acid and talc. Tablets may be uncoated or may be coated by unknown techniques; e.g., to delay disintegration and absorption. Inert diluents and carriers which may be used in capsules include, for example, calcium carbonate, calcium phosphate and kaolin. Suspensions, syrups and elixirs may contain conventional excipients, for example, methyl cellulose, tragacanth, sodium alginate; wetting agents, such as lecithin and polyoxyethylene stearate; and preservatives, e.g., ethyl- p-hydroxybenzoate.

Dosage forms suitable for parenteral administration include solutions, suspensions, dispersions, emulsions and the like. They may also be manufactured in the form of sterile solid compositions which can be dissolved or suspended in sterile injectable medium immediately before use. They may contain suspending or dispersing agents known in the art.

The water solubility of compounds of formula (3) may be improved by modification of the C2' and/or C7 substituents. For instance, water solubility may be increased if X₁ is -OX₆ and R₇ is -OR₂₈, and X₆ and R₂₈ are independently hydrogen or -COGCOR¹ wherein:
G is ethylene, propylene, -CH=CH-, 1,2-cyclohexylene, or 1,2-phenylene;
- R¹ =: OH base, NR²R³, OR³, SR³, OCH₂CONR⁴R⁵, or OH;
- R² =: hydrogen or methyl;
- R³ =: (CH₂)ₙNR⁶R⁷ or (CH₂)ₙN^{⊕}R⁶R⁷R⁸X^{⊖};
- n =: 1 to 3;
- R⁴ =: hydrogen or lower alkyl containing 1 to 4 carbons;
- R⁵ =: hydrogen, lower alkyl containing 1 to 4 carbons, benzyl, hydroxyethyl, CH₂CO₂H, or dimethylaminoethyl;
- R⁶ and R⁷ =: independently selected from lower alkyl containing 1 or 2 carbons or benzyl, or R⁶ and R⁷ together with the nitrogen atom of NR⁶R⁷ forms one of the following rings 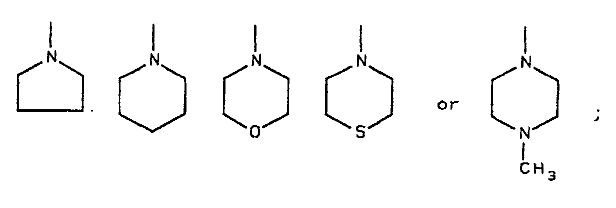
- R⁸ =: lower alkyl containing 1 or 2 carbons, or benzyl;
- X^{e} =: halide;
- base =: NH₃, (HOC₂H₄)₃N, N(CH₃)₃, CH₃N(C₂H₄OH)₂, NH₂(CH₂)₆NH₂, N-methylglucamine, NaOH, or KOH.
The preparation of compounds in which X₁ or X₂ is -COGCOR¹ is set forth in Hangwitz U.S. Patent 4,942,184 which is incorporated herein by reference.

Alternatively, solubility may be increased when X₁ is -OX₆ and X₆ is a radical having the formual -COCX=CHX or -COX-CHX-CHX-SO₂O-M wherein X is hydrogen, alkyl or aryl and M is hydrogen, alkaline metal or an ammonio group as described in Kingston et al., U.S. Patent No. 5,059,699 (incorporated herein by reference).

Taxanes having alternative C9 substituents may be prepared by selectively reducing the C9 keto substituent to yield the corresponding C9 β-hydroxy derivative. The reducing agent is preferably a borohydride and, most preferably, tetrabutylammonium-borohydride (Bu₄NBH₄) or triacetoxy-borohydride.

As illustrated in Reaction Scheme 1, the reaction of baccatin III with Bu₄NBH₄ in methylene chloride yields 9-desoxo-9β-hydroxybaccatin III **5**. After the C7 hydroxy group is protected with the triethylsilyl protecting group, for example, a suitable side chain may be attached to 7-protected-9β-hydroxy derivative **6** as elsewhere described herein. Removal of the remaining protecting groups thus yields 9β-hydroxy-desoxo taxol or other 9β-hydroxytetracylic taxane having a C13 side chain.

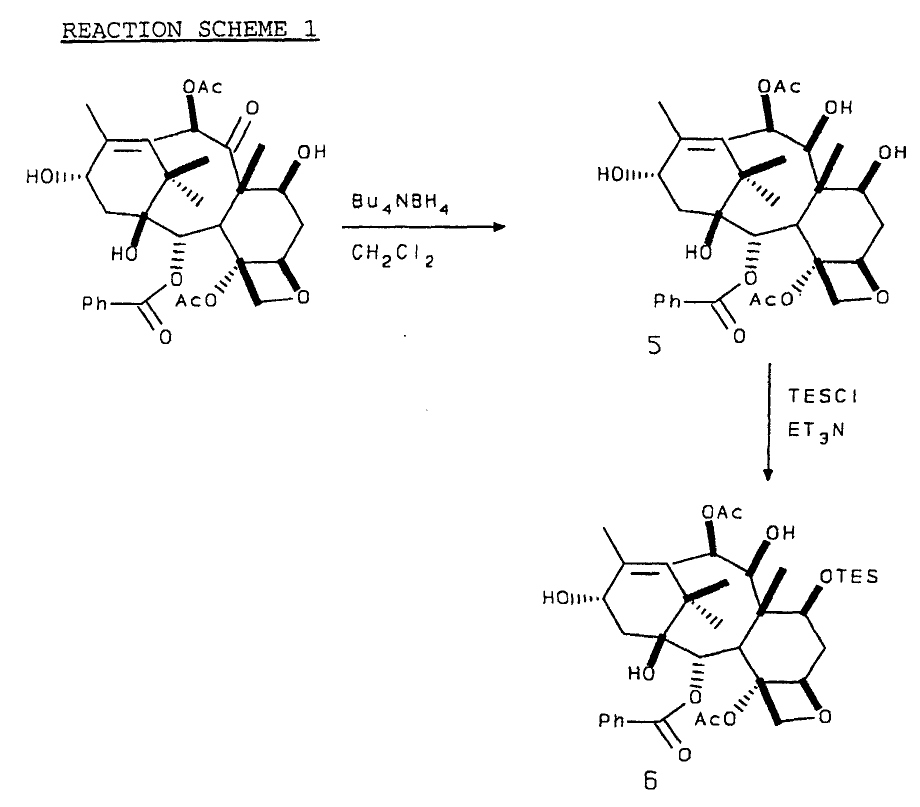

Alternatively, the C13 hydroxy group of 7-protected-9β-hydroxy derivative **6** may be protected with trimethylsilyl or other protecting group which can be selectively removed relative to the C7 hydroxy protecting group as illustrated in Reaction Scheme 2, to enable further selective manipulation of the various substituents of the taxane. For example, reaction of 7,13-protected-9β-hydroxy derivative **7** with KH causes the acetate group to migrate from C10 to C9 and the hydroxy group to migrate from C9 to C10, thereby yielding 10-desacetyl derivative **8.** Protection of the C10 hydroxy group of 10-desacetyl derivative 8 with triethylsilyl yields derivative **9.** Selective removal of the C13 hydroxy protecting group from derivative **9** yields derivative **10** to which a suitable side chain may be attached as described above.

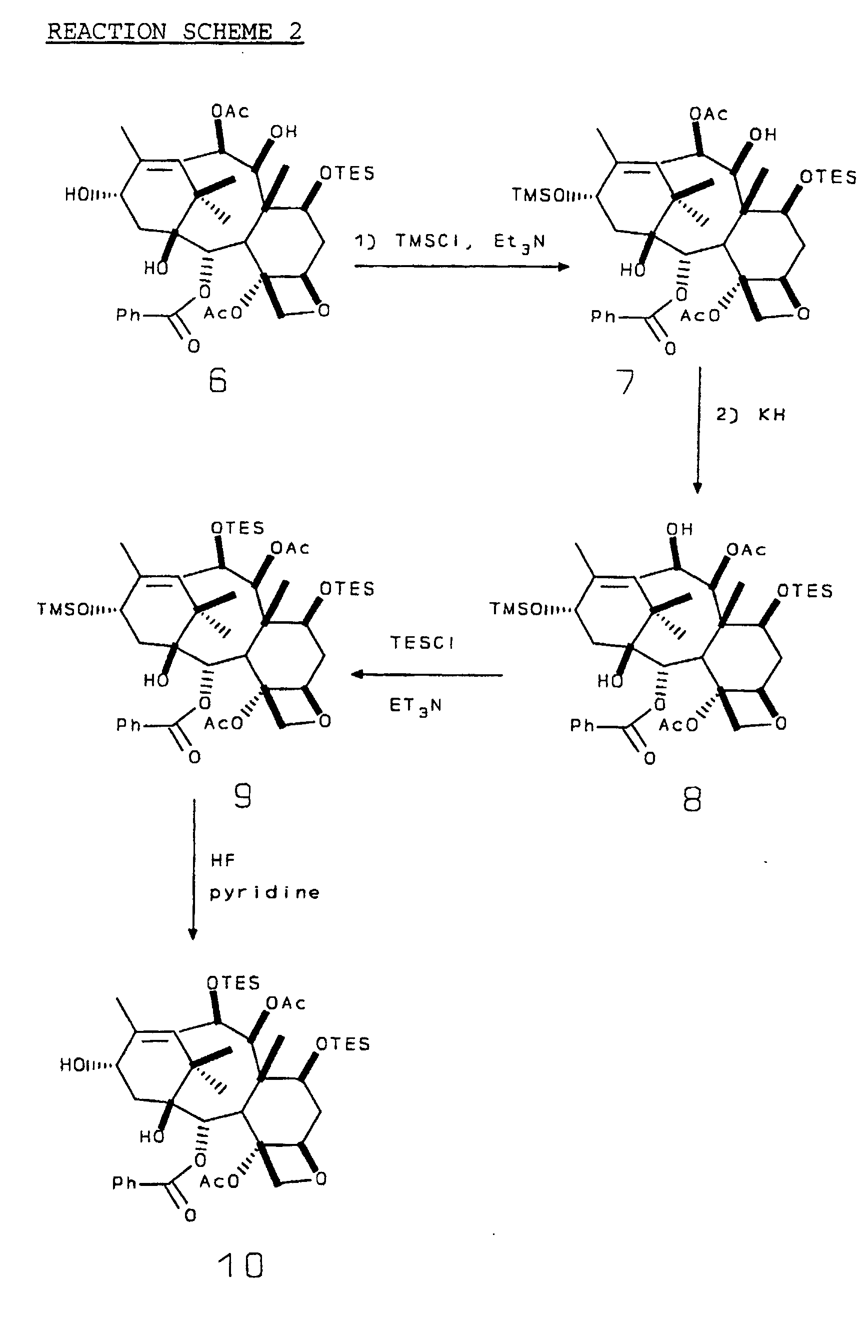

As shown in Reaction Scheme 3, 10-oxo derivative **11** can be provided by oxidation of 10-desacetyl derivative **8.** Thereafter, the C13 hydroxy protecting group can be selectively removed followed by attachment of a side chain as described above to yield 9-acetoxy-10-oxo-taxol or other 9-acetoxy-10-oxotetracylic taxanes having a C13 side chain. Alternatively, the C9 acetate group can be selectively removed by reduction of 10-oxo derivative **11** with a reducing agent such as samarium diiodide to yield 9-desoxo-10-oxo derivative **12** from which the C13 hydroxy protecting group can be selectively removed followed by attachment of a side chain as described above to yield 9-desoxo-10-oxo-taxol or other 9-desoxo-10-oxotetracylic taxanes having a C13 side chain.

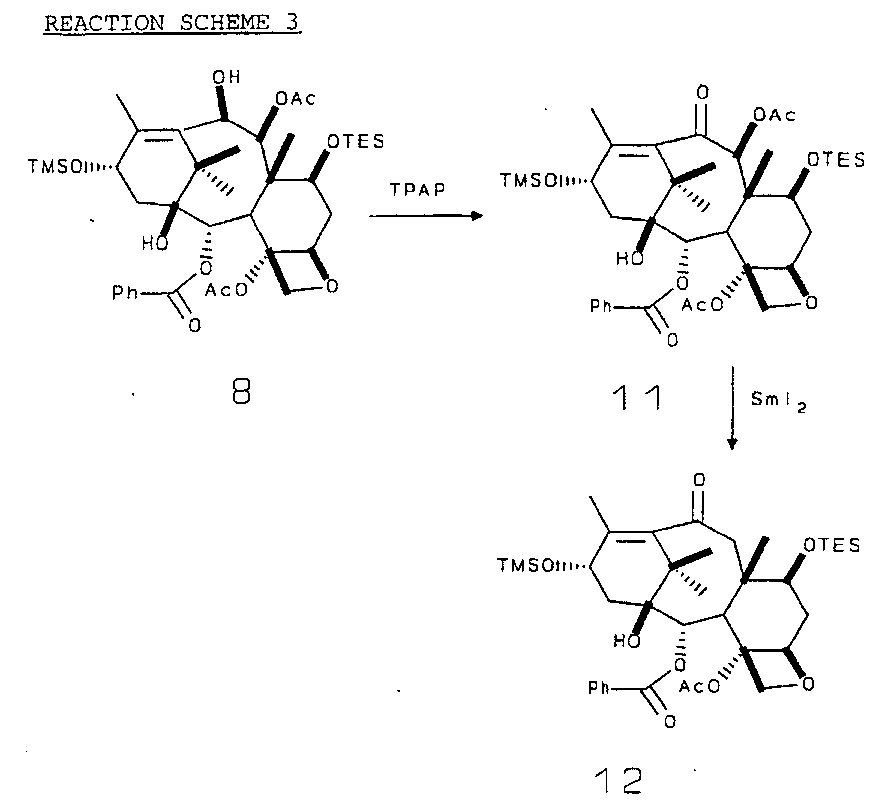

Reaction Scheme 4 illustrates a reaction in which 10'-DAB is reduced to yield pentaol **13.** The C7 and C10 hydroxyl groups of pentaol **13** can then be selectively protected with the triethylsilyl or another protecting group to produce triol **14** to which a C13 side chain can be attached as described above or, alternatively, after further modification of the tetracylic substituents.

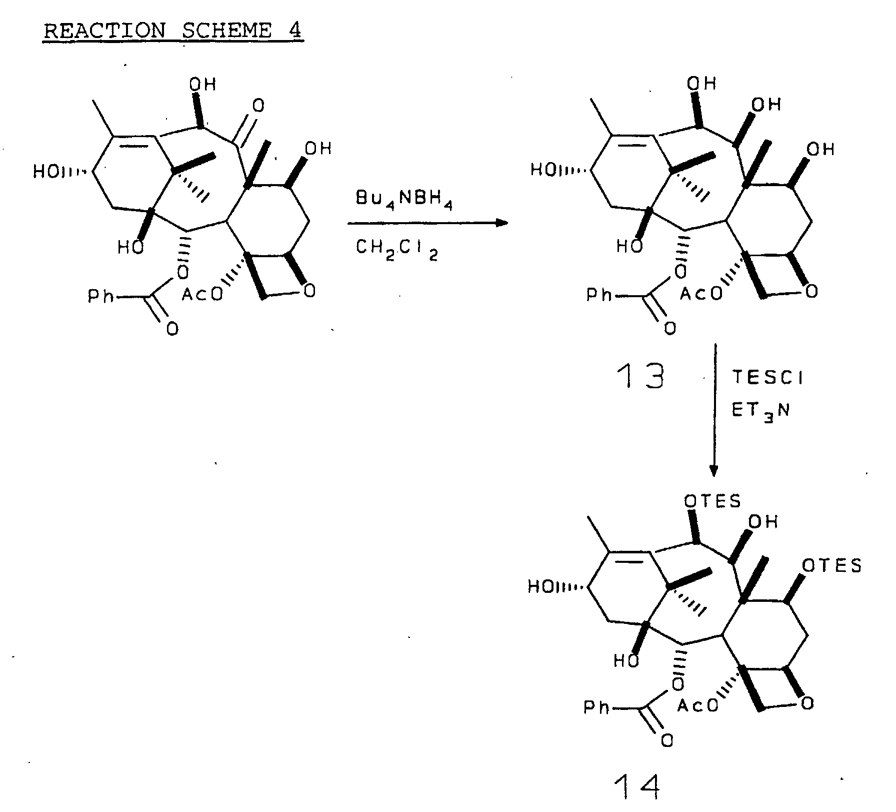

Taxanes having C9 and/or C10 acyloxy substituents other than acetate can be prepared using 10-DAB as a starting material as illustrated in Reaction Scheme 5. Reaction of 10-DAB with triethylsilyl chloride in pyridine yields 7-protected 10-DAB **15.** The C10 hydroxy substituent of 7-protected 10-DAB **15** may then be readily acylated with any standard acylating agent to yield derivative **16** having a new C10 acyloxy substituent. Selective reduction of the C9 keto substituent of derivative **16** yields 9β-hydroxy derivative **17** to which a C13 side chain may be attached. Alternatively, the C10 and C9 groups can be caused to migrate as set forth in Reaction Scheme **2**, above.

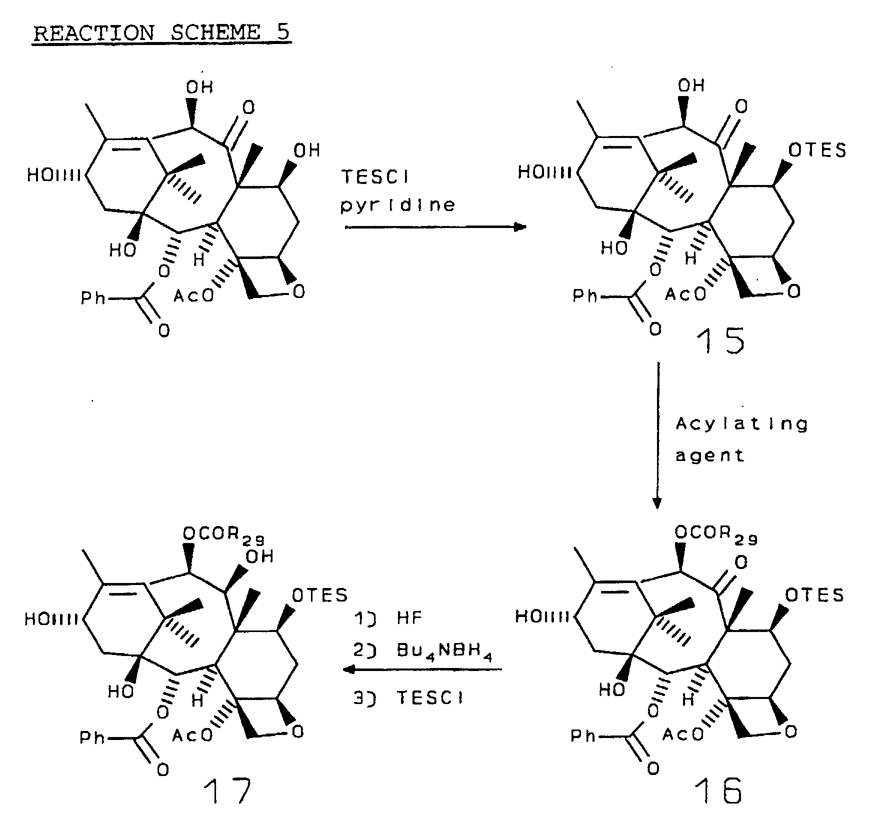

Taxanes having alternative C2 and/or C4 esters can be prepared using baccatin III and 10-DAB as starting materials. The C2 and/or C4 esters of baccatin III and 10-DAB can be selectively reduced to the corresponding alcohol(s) using reducing agents such as LAH or Red-Al, and new esters can thereafter be substituted using standard acylating agents such as anhydrides and acid chlorides in combination with an amine such as pyridine, triethylamine, DMAP, or diisopropyl ethyl amine. Alternatively, the C2 and/or C4 alcohols may be converted to new C2 and/or C4 esters through formation of the corresponding alkoxide by treatment of the alcohol with a suitable base such as LDA followed by an acylating agent such as an acid chloride.

Baccatin III and 10-DAB analogs having different substituents at C2 and/or C4 can be prepared as set forth in Reaction Schemes 6-10. To simplify the description, 10-DAB is used as the starting material. It should be understood, however, that baccatin III derivatives or analogs may be produced using the same series of reactions (except for the protection of the C10 hydroxy group) by simply replacing 10-DAB with baccatin III as the starting material. Derivatives of the baccatin III and 10-DAB analogs having different substituents at C10 and at least one other position, for instance C1, C2, C4, C7, C9 and C13, can then be prepared by carrying out any of the other reactions described herein and any others which are within the level of skill in the art.

In Reaction Scheme 6, protected 10-DAB **3** is converted to the triol **18** with lithium aluminum hydride. Triol **18** is then converted to the corresponding C4 ester using Cl₂CO in pyridine followed by a nucleophilic agent (e.g., Grignard reagents or alkyllithium reagents).

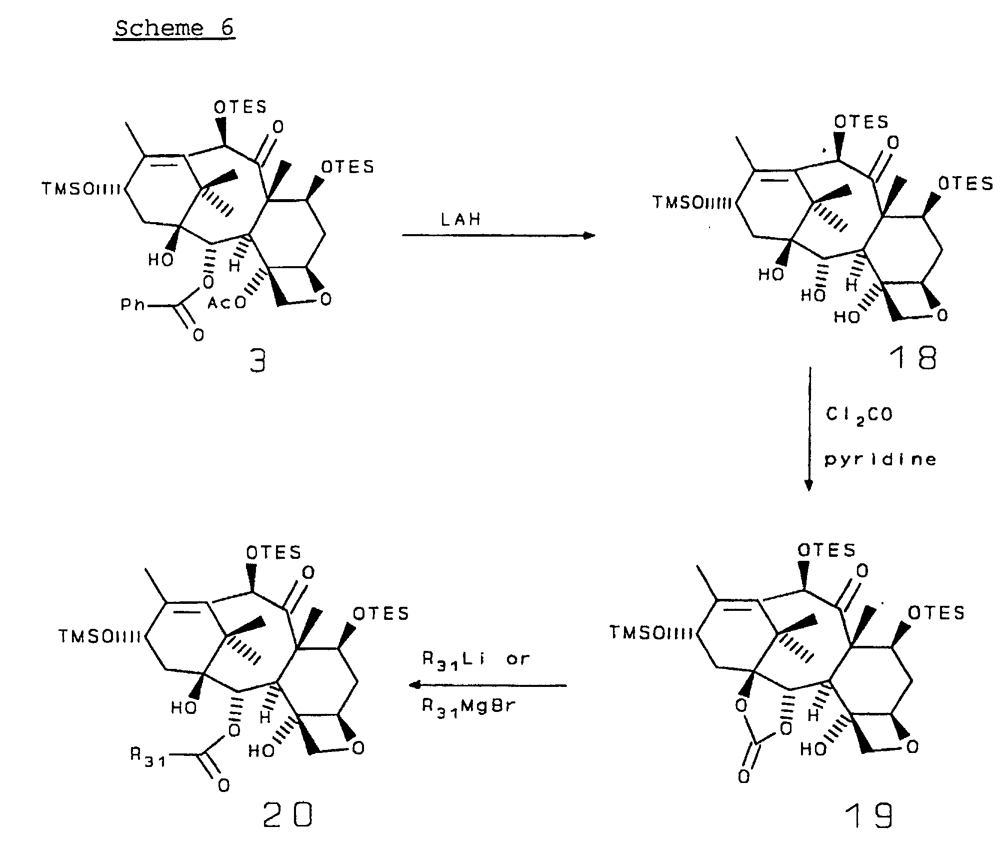

Deprotonation of triol **18** with LDA followed by introduction of an acid chloride selectively gives the C4 ester. For example, when acetyl chloride was used, triol **18** was converted to 1,2 diol **4** as set forth in Reaction Scheme 7.

Triol **18** can also readily be converted to the 1,2 carbonate **19**. Acetylation of carbonate **19** under vigorous standard conditions provides carbonate **21** as described in Reaction Scheme 8; addition of alkyllithiums or Grignard reagents to carbonate **19** provides the C2 ester having a free hydroxyl group at C4 as set forth in Reaction Scheme 6.

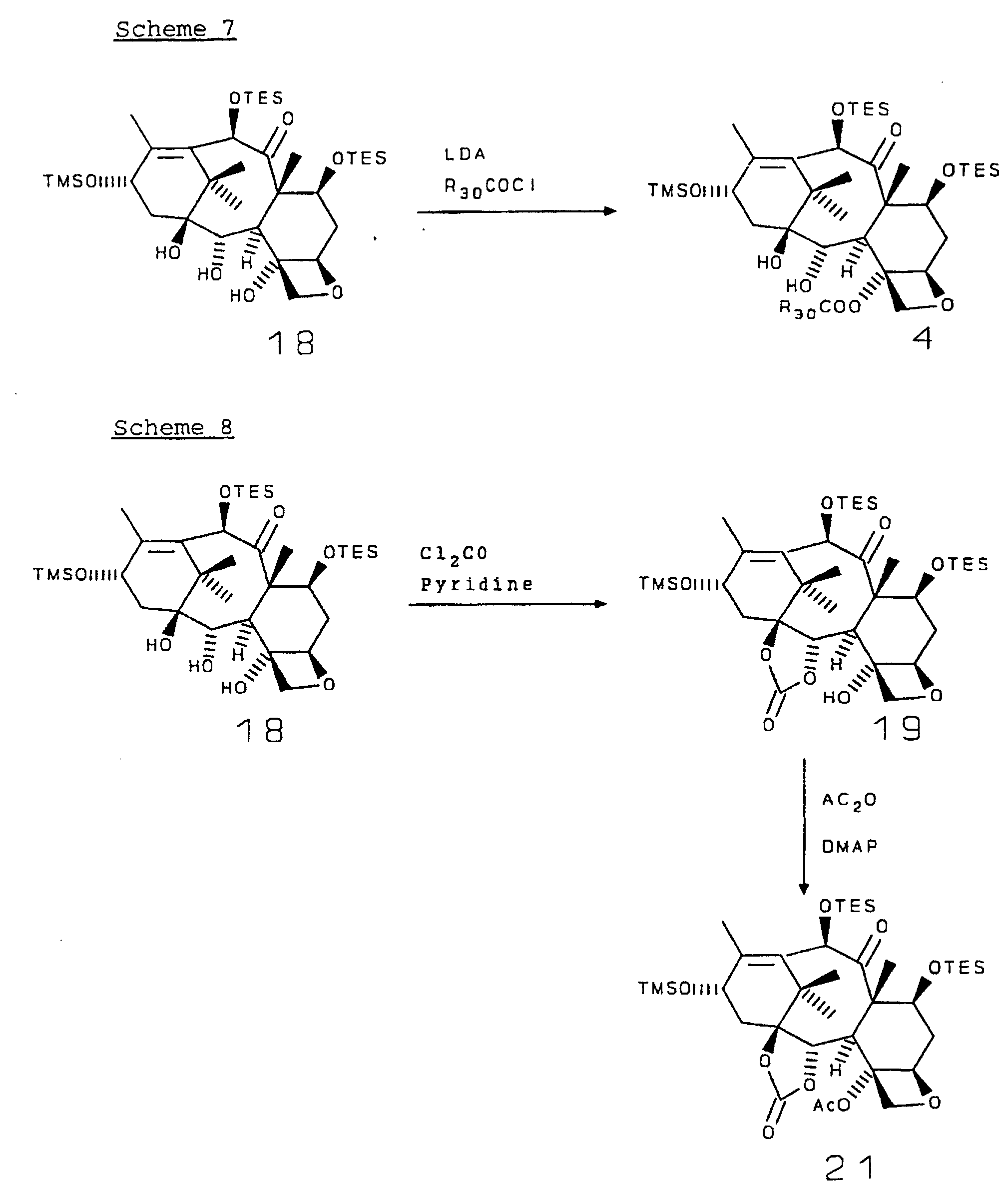

As set forth in Reaction Scheme 9, other C4 substituents can be provided by reacting carbonate **19** with an acid chloride and a tertiary amine to yield carbonate **22** which is then reacted with alkyllithiums or Grignard reagents to provide 10-DAB derivatives having new substituents at C2.

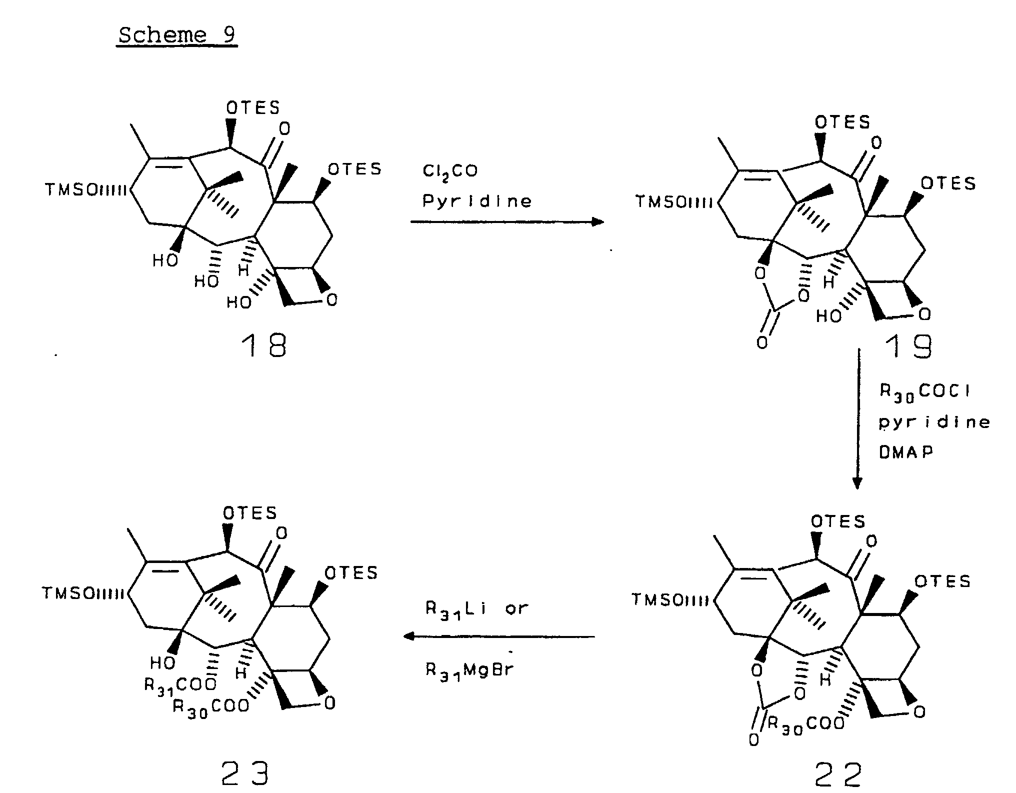

Alternatively, baccatin III may be used as a starting material and reacted as shown in Reaction Scheme 10. After being protected at C7 and C13, baccatin III is reduced with LAH to produce 1,2,4,10 tetraol **24.** Tetraol **24** is converted to carbonate **25** using Cl₂CO and pyridine, and carbonate **25** is acylated at C10 with an acid chloride and pyridine to produce carbonate **26** (as shown) or with acetic anhydride and pyridine (not shown). Acetylation of carbonate **26** under vigorous standard conditions provides carbonate **27** which is then reacted with alkyl lithiums to provide the baccatin III derivatives having new substituents at C2 and C10.

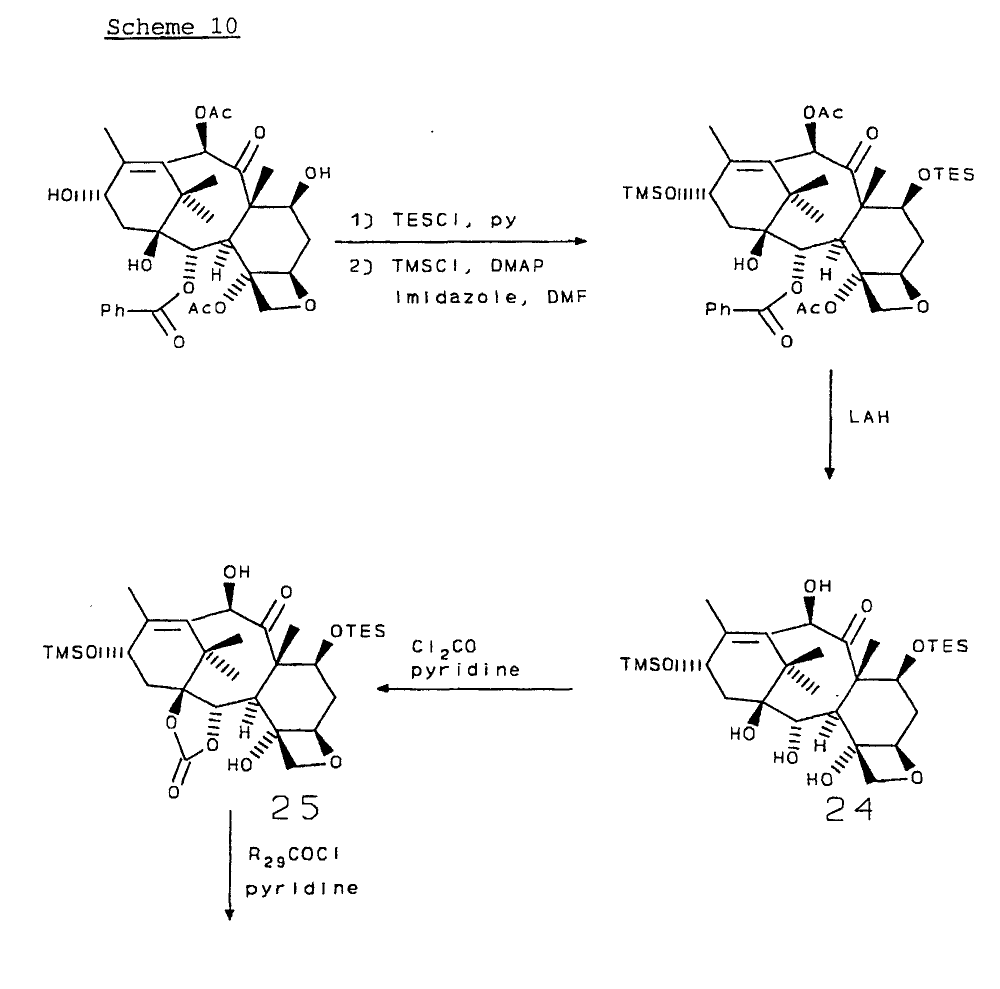

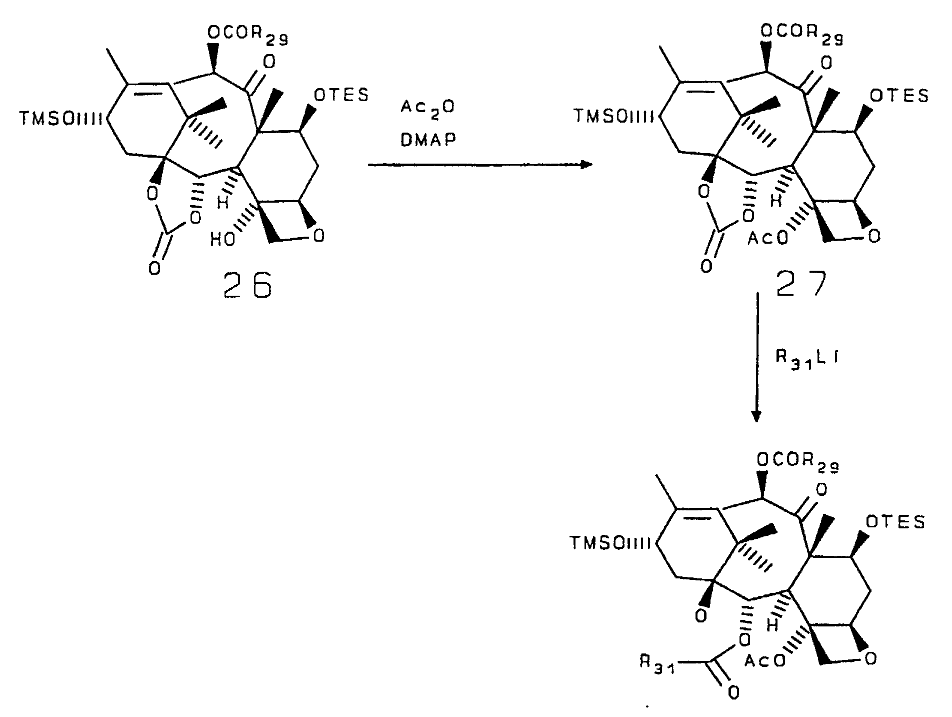

10-desacetoxy derivatives of baccatin III and 10-desoxy derivatives of 10-DAB may be prepared by reacting baccatin III or 10-DAB (or their derivatives) with samarium diiodide. Reaction between the tetracyclic taxane having a C10 leaving group and samarium diiodide may be carried out at 0°C in a solvent such as tetrahydrofuran. Advantageously, the samarium diiodide selectively abstracts the C10 leaving group; C13 side chains and other substituents on the tetracyclic nucleus remain undisturbed. Thereafter, the C9 keto substituent may be reduced to provide the corresponding 9-desoxo-9β-hydroxy-10-desacetyoxy or 10-desoxy derivatives as otherwise described herein.

C7 dihydro and other C7 substituted taxanes can be prepared as set forth in Reaction Schemes 11, 12 and 12a.

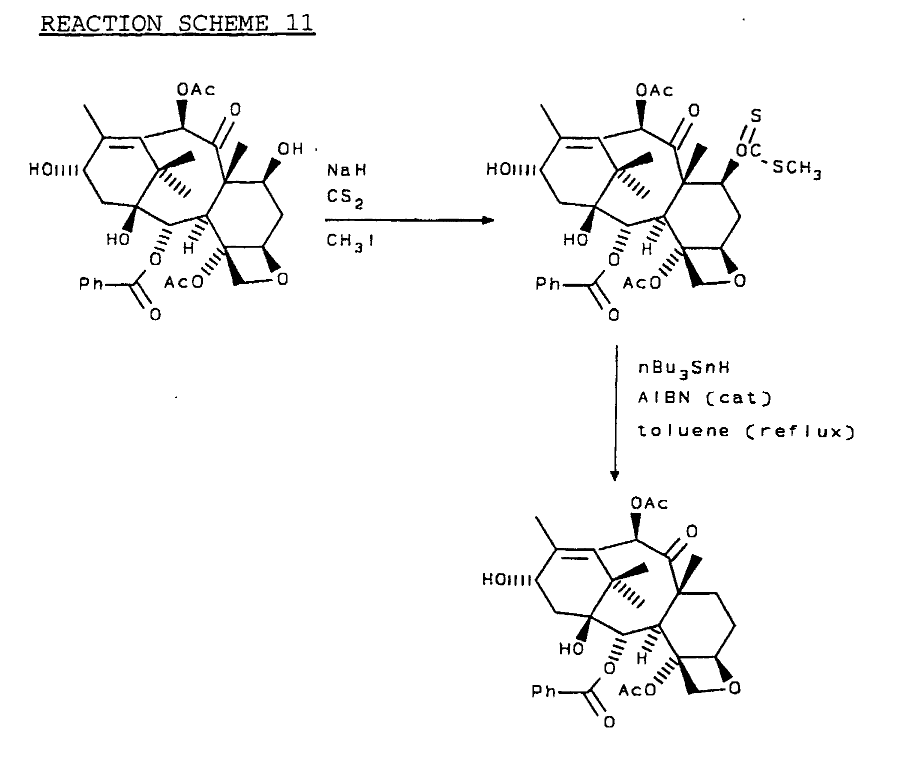

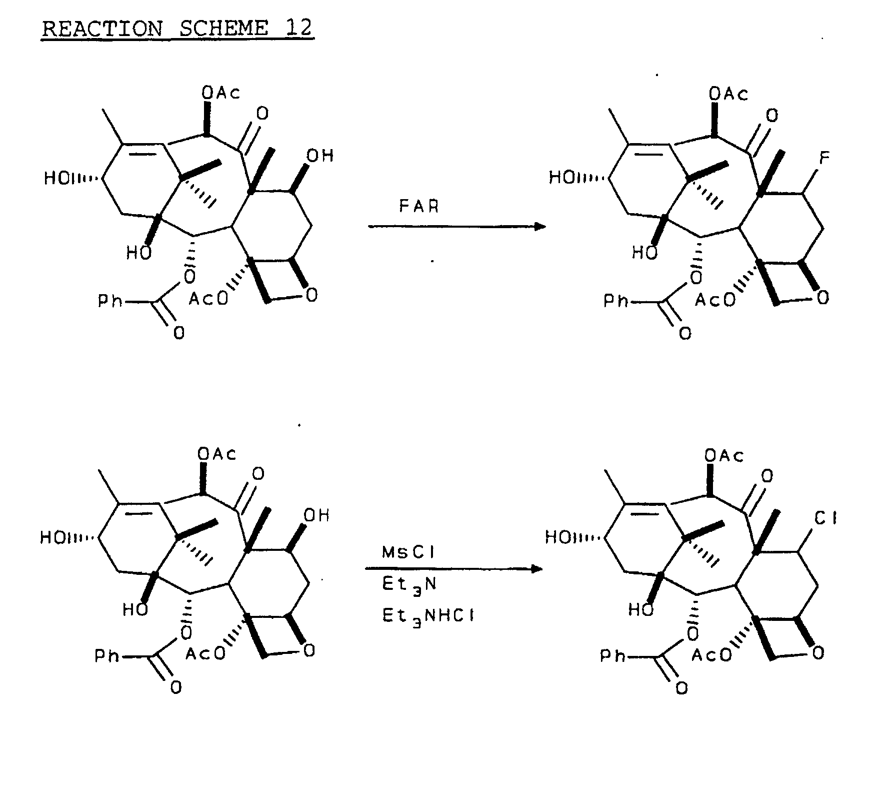

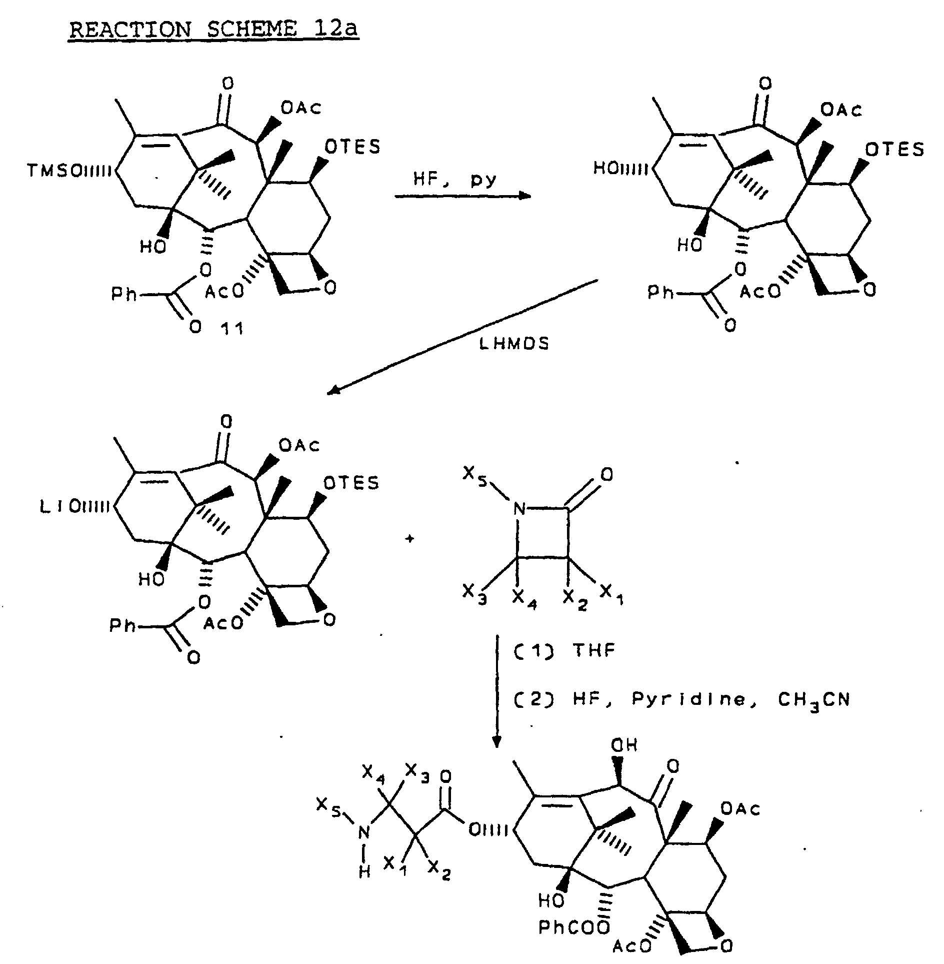

As shown in Reaction Scheme 12, Baccatin III may be converted into 7-fluoro baccatin III by treatment with FAR (or, alternatively, diethylaminosulfur trifluoride ("DAST")) at room temperature in THF solution. Other baccatin derivatives with a free C7 hydroxyl group behave similarly. Alternatively, 7-chloro baccatin III can be prepared by treatment of baccatin III with methane sulfonyl chloride and triethylamine in methylene chloride solution containing an excess of triethylamine hydro-chloride.

A wide variety of tricyclic taxanes are naturally occurring, and through manipulations analogous to those described herein, an appropriate side chain can be attached to the C13 oxygen of these substances. Alternatively, as shown in Reaction Scheme 13, 7-O-triethylsilyl baccatin III can be converted to a tricyclic taxane through the action of trimethyloxonium tetrafluoroborate in methylene chloride solution. The product diol then reacts with lead tetraacetate to provide the corresponding C4 ketone.

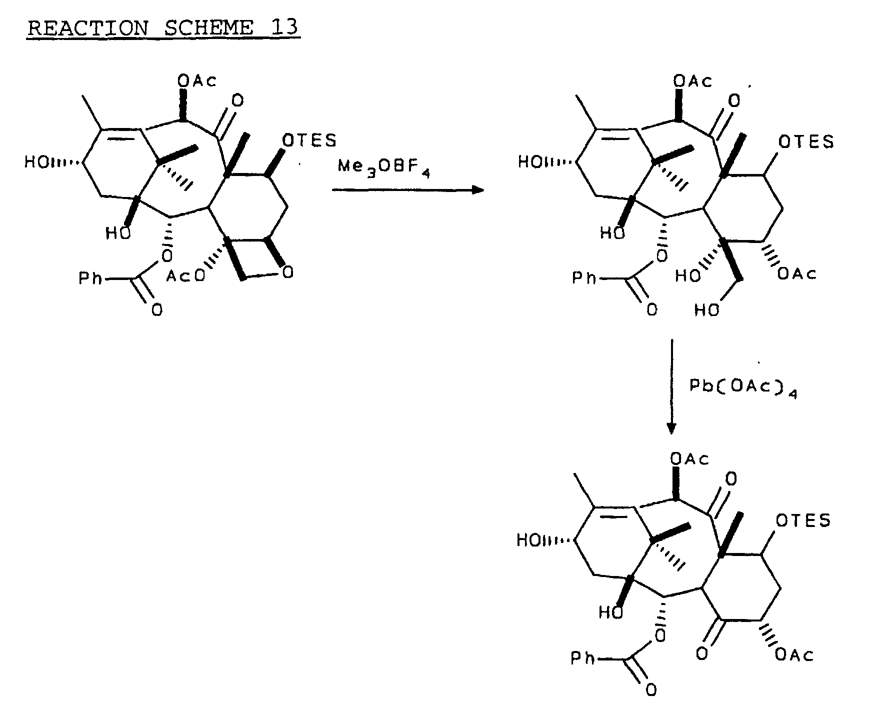

Recently a hydroxylated taxane (14-hydroxy-10-deacetylbaccatin III) has been discovered in an extract of yew needles (C&EN, p 36-37, April 12, 1993). Derivatives of this hydroxylated taxane having the various C2, C4, etc. functional groups described above may also be prepared by using this hydroxylated taxane. In addition, the C14 hydroxy group together with the C1 hydroxy group of 10-DAB can be converted to a 1,2-carbonate as described in C&EN or it may be converted to a variety of esters or other functional groups as otherwise described herein in connection with the C2, C4, C7, C9, C10 and C13 substituents.

The following examples are provided to more fully illustrate the invention.

### EXAMPLE 1 (COMPARATIVE)

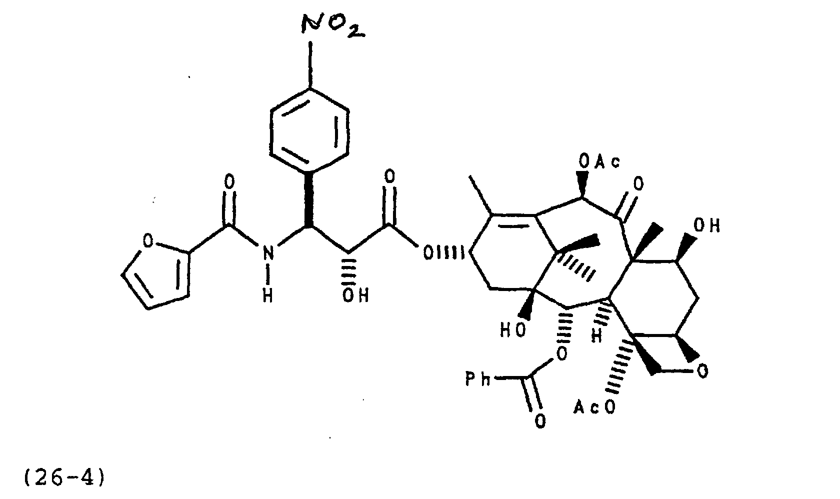

### Preparation of N-debenzoyl-N-(furoyl)-3'-desphenyl-3'-(4-nitrophenyl) taxol.

To a solution of 7-triethylsilyl baccatin III (200 mg, 0.286 mmol) in 2 mL of THF at -45 °C was added dropwise 0.174 mL of a 1.63M solution of nBuLi in hexane. After 0.5 h at -45 °C, a solution of cis-1-(furoyl)-3-triethylsilyloxy-4-(4-nitrophenyl)azetidin-2-one (596 mg, 1.43 mmol) in 2 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 320 mg of a mixture containing (2'R,3'S)-2',7-(bis)triethylsilyl-N-debenzoyl-N-(furoyl)-3'-desphenyl-3'-(4-nitrophenyl) taxol and a small amount of the (2'S,3'R) isomer.

To a solution of 320 mg (0.286 mmol) of the mixture obtained from the previous reaction in 18 mL of acetonitrile and 0.93 mL of pyridine at 0 °C was added 2.8 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 254 mg of material which was purified by flash chromatography to give 187 mg (74%)N-debenzoyl-N-(furoyl)-3'-desphenyl-3'-(4-nitrophenyl) taxol, which was recrystallized from methanol/water.
m.p.184-185 °C; [α]²⁵_{Na}-60.0° (c 0.006, CHCl₃).
¹H NMR (CDCl₃, 300 MHz) δ 8.26 (d, J = 8.79 Hz, 2H, Ar-NO₂), 8.12 (d, J=7.2 Hz, 2H, benzoate ortho), 7.68 (d, J=8.8 Hz 2H, benzamide ortho), 7.7-7.47 (m, 6 H, aromatic), 7.3 (d, J = 9.3 Hz, 1H, NH), 7.02(d, J=3.3 Hz, 1H, furyl), 6.48(dd, J=3.3 Hz, 1.65 Hz, 1H, furyl), 6.27 (s, 1H Hz, H10), 6.26 (dd, J = 8.5, 8.5 Hz, 1H, H13), 5.87 (dd, J = 8.8, 1.65 Hz, 1H, H3'), 5.65 (d, J = 6.6 Hz, 1H, H2β), 4.93 (d, J = 8.2 Hz, 1H, H5), 4.79 (dd, J = 2.7, 1.4 Hz, 1H, H2'), 4.38 (m, 1H, H7), 4.29 (d, J = 8.4 Hz, 1H, H20α), 4.18 (d, J = 8.4 Hz, 1H, H20β), 3.97 (d, J=3.3 Hz, 1H, 2'OH), 3.79 (d, J = 6.6 Hz, 1H, H3), 2.5 (m, 1H, H6α), 2.4(m, 1H, 7OH), 2.38 (s, 3H, 4Ac), 2.27 (m, 2H, H14), 2.22 (s, 3H, 10Ac), 1.88 (m, 1H, H6β), 1.81 (br s, 3H, Me18), 1.78 (s, 1H, 1OH), 1.68 (s, 3H, Me19), 1.21 (s, 3H, Me17), 1.13(s, 3H, Me16).

### EXAMPLES 2-43 (COMPARATIVE)

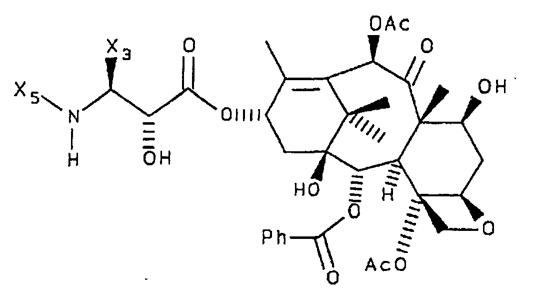

Using the procedure set forth in Example 1 (except for the substituents of azetidin-2-one and the amounts of the reactants) a series of compounds were prepared having the structure shown above in which X₃ and X₅ are as shown in the following table. The structures were confirmed by NMR.

**TABLE 1**

| **Example** | **Compound #** | **X**_{**3**} | **X**_{**5**} |
|---|---|---|---|
| 2 | 30-2 | phenyl | 2-furoyl |
| 3 | 31-1 | phenyl | 2-thienoyl |
| 4 | 31-4 | 2-furyl | t-butoxycarbonyl |
| 5 | 32-1 | 2-furyl | ethoxycarbonyl |
| 6 | 33-1 | 2-thienyl | cyclohexyloxycarbonyl |
| 7 | 34-2 | 2-thienyl | t-butoxycarbonyl |
| 8 | 34-3 | 2-thienyl | 2-thienoyl |
| 9 | 34-4 | 2-thienyl | 2-furoyl |
| 10 | 35-1 | 2-thienyl | n-butoxycarbonyl |
| 11 | 35-2 | 2-thienyl | allyloxycarbonyl |
| 12 | 35-4 | 2-thienyl | diethylcarbamyl |
| 13 | 36-3 | 2-furyl | 4-methylbenzoyl |
| 14 | 37-1 | 2-furyl | isobutoxycarbonyl |
| 15 | 37-2 | 2-furyl | butoxycarbonyl |
| 16 | 37-3 | 2-furyl | diethylcarbamyl |
| 17 | 37-4 | 2-furyl | isopropoxycarbonyl |
| 18 | 38-1 | 2-furyl | allyloxycarbonyl |
| 19 | 38-2 | 2-furyl | benzyloxycarbonyl |
| 20 | 38-3 | 2-furyl | diphenylcarbamyl |
| 21 | 39-3 | 2-thienyl | ethoxycarbonyl |
| 22 | 39-4 | 2-thienyl | 3-butynyloxycarbonyl |
| 23 | 40-1 | 2-thienyl | crotyloxycarbonyl |
| 24 | 40-2 | 2-thienyl | 1,3-diethoxy-2-propoxycarbonyl |
| 25 | 40-3 | 2-thienyl | methoxyethoxycarbonyl |
| 26 | 40-4 | 2-thienyl | neopentyloxycarbonyl |
| 27 | 41-1 | 2-thienyl | isopropoxycarbonyl |
| 28 | 41-2 | 2-thienyl | isobutoxycarbonyl |
| 29 | 42-3 | 2-furyl | 2-thienylcarbonyl |
| 30 | 42-4 | 2-furyl | 2-methoxyethoxycarbonyl |
| 31 | 43-1 | 2-furyl | crotyloxycarbonyl |
| 32 | 43-2 | 2-furyl | neopentyloxycarbonyl |
| 33 | 43-3 | 2-furyl | cyclohexyloxycarbonyl |
| 34 | 43-4 | 2-furyl | 1,3-diethoxy-2-propyloxycarbonyl |
| 35 | 44-1 | 2-furyl | 3-butynyloxycarbonyl |
| 36 | 44-2 | 2-furyl | N-methyl-N-phenylcarbamoyl |
| 37 | 44-3 | 2-furyl | N,N-dimethylcarbamoyl |
| 38 | 44-4 | 2-furyl | 4-morpholinocarbonyl |
| 39 | 45-1 | 2-furyl | 2-furoyl |
| 40 | 46-2 | 2-furyl | N-n-propylcarbamoyl |
| 41 | 46-3 | 2-furyl | N-phenylcarbamoyl |
| 42 | 46-4 | 2-thienyl | N-phenylcarbamoyl |
| 43 | 47-1 | 2-thienyl | N-n-propylcarbamoyl |

### EXAMPLE 44 (COMPARATIVE)

The compounds of the preceding examples were in in vitro cytotoxicity activity against human colon carcinoma cells HCT-116 and HCT-116/VM46. The HCT116/VM cells are cells that have been selected for teniposide resistance and express the multidrug resistance phenotype, including resistance to taxol. Cytotoxicity was assessed in HCT116 and HCT VM46 human colon carcinoma cells by XTT (2,3-bis(2-methoxy-4-nitro-5-sulfophenyl)-5-[(phenylamino)carbonyl]-2H-tetrazolium hydroxide) assay (Scudiero et al, "Evaluation of a soluble tetrazolium/formazan assay for cell growth and drug sensitivity in culture using human and other tumor cell lines", Cancer Res. 48:4827-4833, 1988). Cells were plated at 4000 cells/well in 96 well microtiter plates and 24 hours later drugs were added and serial diluted. The cells were incubated at 37°C for 72 hours at which time the tetrazolium dye, XTT, was added. A dehydrogenase enzyme in live cells reduces the XTT to a form that absorbs light at 450 nm which can be quantitated spectrophotometrically. The greater the absorbance the greater the number of live cells. The results are expressed as an IC₅₀ which is the drug concentration required to inhibit cell proliferation (i.e. absorbance at 450 nm) to 50% of that of untreated control cells. The results are presented in Table 2 and are compared to taxol and taxotere. Lower numbers indicate greater activity.

**TABLE 2**

| | | IC₅₀ | |
|---|---|---|---|
| Example | Compound | HCT 116 | HCT VM46 |
| 1 | 26-4 | .002 | .883 |
| 2 | 30-2 | .003 | .300 |
| 3 | 31-1 | .002 | .202 |
| 4 | 31-4 | .001 | .004 |
| 5 | 32-1 | .001 | .032 |
| 6 | 33-1 | .001 | <.017 |
| 7 | 34-2 | .001 | <.018 |
| 8 | 34-3 | .001 | .042 |
| | | .001 | <.023 |
| 9 | 34-4 | .001 | .051 |
| | | .001 | .034 |
| 10 | 35-1 | .001 | <.025 |
| | | .002 | .016 |
| 11 | 35-2 | .004 | .022 |
| 12 | 35-4 | .019 | .581 |
| 13 | 36-3 | .006 | .150 |
| 14 | 37-1 | .004 | .022 |
| | | .001 | .033 |
| | | <.001 | .018 |
| 15 | 37-2 | .005 | <.021 |
| | | .001 | .030 |
| | | .001 | .021 |
| 16 | 37-3 | .016 | 1.10 |
| 17 | 37-4 | .005 | <.015 |
| | | .004 | .008 |
| | | .001 | .008 |
| 18 | 38-1 | .002 | <.031 |
| 19 | 38-2 | .003 | .062 |
| 20 | 38-3 | >.078 | >7.8 |
| 21 | 39-3 | .001 | .019 |
| 22 | 39-4 | .002 | .163 |
| 23 | 40-1 | .001 | .011 |
| 24 | 40-2 | .030 | .597 |
| 25 | 40-3 | .006 | .251 |
| 26 | 40-4 | .022 | .090 |
| 27 | 41-1 | .001 | .008 |
| 28 | 41-2 | .002 | .023 |
| 29 | 42-3 | .002 | .034 |
| 30 | 42-4 | .002 | .216 |
| 31 | 43-1 | .001 | .010 |
| 32 | 43-2 | .001 | .009 |
| 33 | 43-3 | .001 | .009 |
| 34 | 43-4 | .005 | .432 |
| 35 | 44-1 | .001 | .175 |
| 36 | 44-2 | .018 | .785 |
| 37 | 44-3 | .010 | 2.11 |
| 38 | 44-4 | .020 | 3.60 |
| 39 | 45-1 | .001 | .041 |
| 40 | 46-2 | .013 | .781 |
| 41 | 46-3 | .008 | .831 |
| 42 | 46-4 | .005 | .902 |
| 43 | 47-1 | .008 | .810 |
| | taxol | .004 | .536 |
| | | .002 | .313 |
| | taxotere | .007 | .246 |
| | | .003 | .189 |

### EXAMPLES 45-49

Using the procedures set forth in Example 1 (except for the substituents of azetidin-2-one and the protected taxane and the amounts of the reactants) a series of compounds were prepared having the following structure in which X₃, X₁₀, R₂, R₇, R₉ₐ, and R₁₀ₐ are as shown in Table 3. Unless otherwise indicated, R₂ is benzoyloxy, R₇ is hydroxy, R₉ₐ is keto, and R₁₀ₐ is acetoxy. The structures were confirmed by NMR.

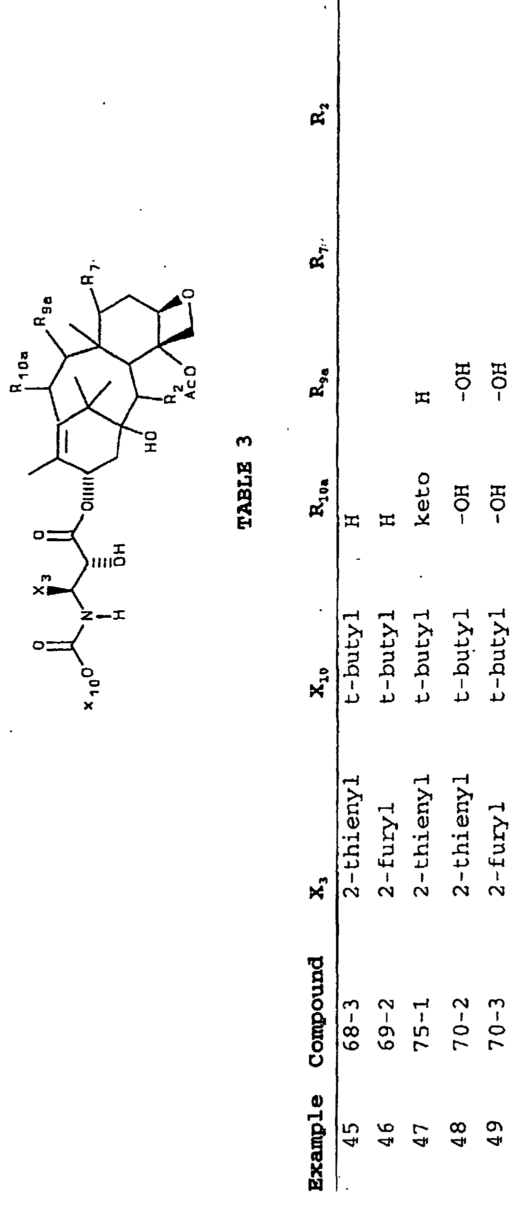

### EXAMPLE 50

The taxanes of the Examples 45-49 were evaluated using the procedures set forth in Example 44. All compounds had an IC₅₀ of less than 0.1, indicating that they are cytotoxically active.

In view of the above, it will be seen that the several objects of the invention are achieved.

As various changes could be made in the above compositions without departing from the scope of the invention, it is intended that all matter contained in the above description be interpreted as illustrative and not in a limiting sense.

## Claims

1. A taxane derivative having the formula: 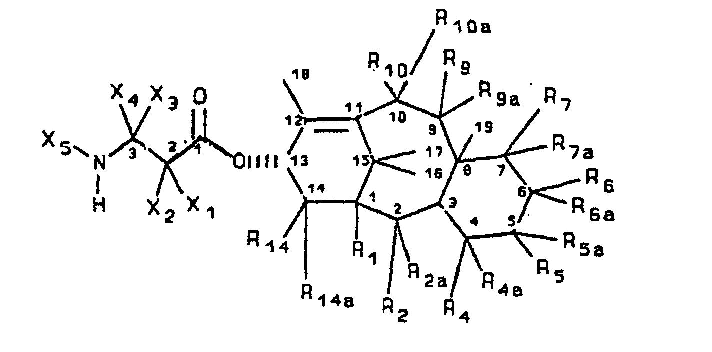 wherein
X₁ is -OX₆, -SX₇, or -NX₈X₉;
X₂ is hydrogen, alkyl, alkenyl, alkynyl, aryl, or heteroaryl;
X₃ and X₄ are independently hydrogen, alkyl, alkenyl, alkynyl, aryl, heteroaryl, acyl or heterosubstituted alkyl, alkenyl, alkynyl, aryl or heteroaryl, provided, however, that X₃ and X₄ are not both acyl;
X₅ is -COX₁₀, -COOX₁₀, -COSX₁₀, -CONX₈X₁₀, or -SO₂X₁₁;
X₆ is hydrogen, alkyl, alkenyl, alkynyl, aryl, heteroaryl, or a hydroxy protecting group;
X₇ is alkyl, alkenyl, alkynyl, aryl, heteroaryl, or sulfhydryl protecting group;
X₈ is hydrogen, alkyl, alkenyl, alkynyl, aryl, heteroaryl, or heterosubstituted alkyl, alkenyl, alkynyl, aryl or heteroaryl;
X₉ is an amino protecting group;
X₁₀ is alkyl, alkenyl, alkynyl, aryl, heteroaryl, or heterosubstituted alkyl, alkenyl, alkynyl, aryl or heteroaryl;
X₁₁ is alkyl, alkenyl, alkynyl, aryl, heteroaryl, -OX₁₀, or -NX₈X₁₄;
X₁₄ is hydrogen, alkyl, alkenyl, alkynyl, aryl, or heteroaryl;
R₁₄ is hydrogen, alkyl, alkenyl, alkynyl, aryl, or heteroaryl, hydroxy, protected hydroxy or together with R₁ forms a carbonate;
R₁₄ₐ is hydrogen, alkyl, alkenyl, alkynyl, aryl, or heteroaryl;
R₁₀ is hydrogen or together with R₁₀ₐ forms an oxo;
R₁₀ₐ is hydrogen, -OCOR₂₉, hydroxy, or protected hydroxy, or together with R₁₀ forms an oxo;
R₉ is hydrogen or together with R₉ₐ forms an oxo;
R₉ₐ is hydrogen, hydroxy, protected hydroxy, acyloxy, or together with R₉ forms an oxo;
R₇ₐ is hydrogen or together with R₇ forms an oxo;
R₇ is hydroxy, or together with R₇ₐ forms an oxo;
R₆ is hydrogen, alkyl, alkenyl, alkynyl, aryl, or heteroaryl, hydroxy, protected hydroxy or together with R₆ₐ forms an oxo;
R₆ₐ is hydrogen, alkyl, alkenyl, alkynyl, aryl, or heteroaryl, hydroxy, protected hydroxy or together with R₆ forms an oxo;
R₅ is hydrogen or together with R₅ₐ forms an oxo;
R₅ₐ is hydrogen, hydroxy, protected hydroxy, acyloxy, together with R₅ forms an oxo, or together with R₄ and the carbon atoms to which they are attached form an oxetane ring;
R₄ is hydrogen, together with R₄ₐ forms an oxo or together with R₅ₐ and the carbon atoms to which they are attached form an oxetane ring;
R₄ₐ is hydrogen, alkyl, alkenyl, alkynyl, aryl, heteroaryl, cyano, hydroxy, -OCOR₃₀, or together with R₄ forms an oxo, oxirane or methylene;
R₂ is hydrogen, hydroxy, or -OCOR₃₁;
R₂ₐ is hydrogen or taken together with R₂ forms an oxo;
R₁ is hydrogen, hydroxy, protected hydroxy;
R₂₉, R₃₀, and R₃₁ are independently hydrogen, alkyl, alkenyl, alkynyl, monocyclic aryl or monocyclic heteroaryl;
provided, however, that at least one of X₃, X₄ and X₁₀ is furyl or thienyl; and either (i) R₁₀ₐ is hydrogen or together with R₁₀ forms an oxo; (ii) R₉ₐ is hydrogen, hydroxy, protected hydroxy, or acyloxy; (iii) R₇ together with R₇ₐ forms an oxo; (iv) R₄ₐ is hydrogen, alkyl, alkenyl, alkynyl, aryl, heteroaryl, cyano, hydroxy, -OCOR₃₀ wherein R₃₀ is other than methyl, or together with R₄ forms an oxo, oxirane or methylene; (v) R₂ is hydrogen, hydroxy, or -OCOR₃₁ wherein R₃₁ is other than phenyl; (vi) R₁ is other than hydroxy or (vii) R₁₄ is other than hydrogen.

2. A taxane derivative according to claim 1, wherein R₁₀ₐ is hydrogen or together with R₁₀ forms an oxo or R₉ₐ is hydrogen, hydroxy, protected hydroxy, or acyloxy.

3. A taxane derivative according to claim 1 or claim 2, wherein R₄ₐ is hydrogen, alkyl, alkenyl, alkynyl, aryl, heteroaryl, cyano, hydroxy, -OCOR₃₀ wherein R₃₀ is other than methyl, or together with R₄ forms an oxo, oxirane or methylene or R₂ is hydrogen, hydroxy, or -OCOR₃₁ wherein R₃₁ is other than phenyl.

4. A taxane derivative according to any one of claims 1 to 3, wherein X₃ or X₄ is thienyl, X₅ is -COOX₁₀, and X₁₀ is other than phenyl and alkoxy.

5. A taxane derivative according to any one of claims 1 to 4, wherein X₅ is -COX₁₀, X₁₀ is furyl or thienyl and neither of X₃ or X₄ are phenyl or p-nitro substituted phenyl.

6. A taxane derivative according to any one of claims 1 to 5, wherein R₁ is other than hydroxy or R₁₄ is other than hydrogen.

## Patentansprüche

1. Taxan-Derivat mit der Formel 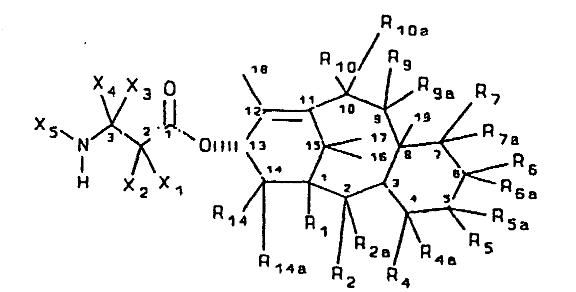 worin
X₁ -OX₆, -SX₇ oder -NX₈ X₉ ist;
X₂ Wasserstoff, Alkyl, Alkenyl, Alkynyl, Aryl oder Heteroaryl ist;
X₃ und X₄ unabhängig Wasserstoff, Alkyl, Alkenyl, Alkynyl, Aryl, Hetroaryl, Acyl oder heterosubstituiertes Alkyl, Alkenyl, Alkynyl, Aryl oder Hetroaryl sind, vorausgesetzt jedoch, daß X₃ und X₄ nicht beide Acyl sind;
X₅ -COX₁₀ , -COOX₁₀ , -COSX₁₀ , -CONX₈X₁₀ oder -SO₂X₁₁ ist;
X₆ Wasserstoff, Alkyl, Alkenyl, Alkynyl, Aryl, Heteroaryl oder eine Hydroxy-Schutzgruppe ist;
X₇ Alkyl, Alkenyl, Alkynyl, Aryl, Heteroaryl oder eine Sulfhydryl-Schutzgruppe ist;
X₈ Wasserstoff, Alkyl, Alkenyl, Alkynyl, Aryl, Heteroaryl oder heterosubstituiertes Alkyl, Alkenyl, Alkynyl, Aryl oder Heteroaryl ist;
X₉ eine Amino-Schutzgruppe ist;
X₁₀ Alkyl, Alkenyl, Alkynyl, Aryl, Heteroaryl oder heterosubstituiertes Alkyl, Alkenyl, Alkynyl, Aryl oder Heteroaryl ist;
X₁₁ Alkyl, Alkenyl, Alkynyl, Aryl, Heteroaryl, -OX₁₀ oder -NX₈X₁₄ ist;
X₁₄ Wasserstoff, Alkyl, Alkenyl, Alkynyl, Aryl oder Heteroaryl ist,
R₁₄ Wasserstoff, Alkyl, Alkenyl, Alkynyl, Aryl oder Heteroaryl, Hydroxy, geschütztes Hydroxy ist oder zusammen mit R₁ ein Carbonat bildet;
R₁₄ₐ Wasserstoff, Alkyl, Alkenyl, Alkynyl, Aryl oder Heteroaryl ist;
R₁₀ Wasserstoff ist oder zusammen mit R₁₀ₐ ein Oxo bildet;
R₁₀ₐ Wasserstoff, -OCOR₂₉, Hydroxy oder geschütztes Hydroxy ist oder zusammen mit R₁₀ ein Oxo bildet;
R₉ Wasserstoff ist oder zusammen mit R₉ₐ ein Oxo bildet;
R₉ₐ Wasserstoff, Hydroxy, geschütztes Hydroxy, Acyloxy ist oder zusammen mit R₉ ein Oxo bildet;
R₇ₐ Wasserstoff ist oder zusammen mit R₇ ein Oxo bildet;
R₇ Hydroxy ist oder zusammen mit R₇ₐ ein Oxo bildet;
R₆ Wasserstoff, Alkyl, Alkenyl, Alkynyl, Aryl oder Heteroaryl, Hydroxy, geschütztes Hydroxy ist oder zusammen mit R₆ₐ ein Oxo bildet;
R₆ₐ Wasserstoff, Alkyl, Alkenyl, Alkynyl, Aryl oder Heteroaryl, Hydroxy, geschütztes Hydroxy ist oder zusammen mit R₆ ein Oxo bildet;
R₅ Wasserstoff ist oder zusammen mit R₅ₐ ein Oxo bildet;
R₅ₐ Wasserstoff, Hydroxy, geschütztes Hydroxy, Acyloxy ist, zusammen mit R₅ ein Oxo bildet oder zusammen mit R₄ und den Kohlenstoffatomen, an denen sie hängen, einen Oxetanring bildet;
R₄ Wasserstoff ist, zusammen mit R₄ₐ ein Oxo bildet oder zusammen mit R₅ₐ und den Kohlenstoffatomen, an denen sie hängen, einen Oxetanring bildet;
R₄ₐ Wasserstoff, Alkyl, Alkenyl, Alkynyl, Aryl, Heteroaryl, Cyano, Hydroxy, -OCOR₃₀ ist oder zusammen mit R₄ ein Oxo, Oxiran oder Methylen bildet;
R₂ Wasserstoff, Hydroxy oder -OCOR₃₁ ist;
R₂ₐ Wasserstoff ist oder zusammen mit R₂ ein Oxo bildet;
R₁ Wasserstoff, Hydroxy, geschütztes Hydroxy ist;
R₂₉, R₃₀ und R₃₁ unabhängig Wasserstoff, Alkyl, Alkenyl, Alkynyl, monocyclisches Aryl oder monocyclisches Heteroaryl sind;
vorausgesetzt jedoch, daß wenigstens eins von X₃, X₄ und X₁₀ Furyl oder Thienyl ist; und entweder (i) R₁₀ₐ Wasserstoff ist oder zusammen mit R₁₀ ein Oxo bildet, (ii) R₉ₐ Wasserstoff, Hydroxy, geschütztes Hydroxy oder Acyloxy ist, (iii) R₇ zusammen mit R₇ₐ ein Oxo bildet, (iv) R₄₀ Wasserstoff, Alkyl, Alkenyl, Alkynyl, Aryl, Heteroaryl, Cyano, Hydroxy, -OCOR₃₀ ist, wobei R₃₀ von Methyl verschieden ist oder zusammen mit R₄ ein Oxo, Oxiran oder Methylen bildet, (v) R₂ Wasserstoff, Hydroxy oder -OCOR₃₁ ist, wobei R₃₁ von Phenyl verschieden ist, (vi) R₁ von Hydroxy verschieden ist oder (vii) R₁₄ von Wasserstoff verschieden ist.

2. Taxan-Derivat nach Anspruch 1, bei dem R₁₀ₐ Wasserstoff ist oder zusammen mit R₁₀ein Oxo bildet oder R₉ₐ Wasserstoff, Hydroxy, geschütztes Hydroxy oder Acyloxy ist.

3. Taxan-Derivat nach Anspruch 1 oder Anspruch 2, bei dem R₄ₐ Wasserstoff, Alkyl, Alkenyl, Alkynyl, Aryl, Heteroaryl, Cyano, Hydroxy, -OCOR₃₀ ist, worin R₃₀ von Methyl verschieden ist, oder zusammen mit R₄ ein Oxo, Oxiran oder Methylen bildet oder R₂ Wasserstoff, Hydroxy oder -OCOR₃₁ ist, worin R₃₁ von Phenyl verschieden ist.

4. Taxan-Derivat nach einem der Ansprüche 1 bis 3, bei dem X₃ oder X₄Thienyl ist, X₅ -COOX₁₀ ist und X₁₀ von Phenyl und Alkoxy verschieden ist.

5. Taxan-Derivat nach einem der Ansprüche 1 bis 4, bei dem X₅ -COX₁₀ ist, X₁₀ Furyl oder Thienyl ist und weder X₃ noch X₄ Phenyl oder p-nitrosubstituiertes Phenyl ist.

6. Taxan-Derivat nach einem der Ansprüche 1 bis 5, bei dem R₁ von Hydroxy verschieden ist oder R₁₄ von Wasserstoff verschieden ist.

## Revendications

1. Dérivé de taxane de formule: 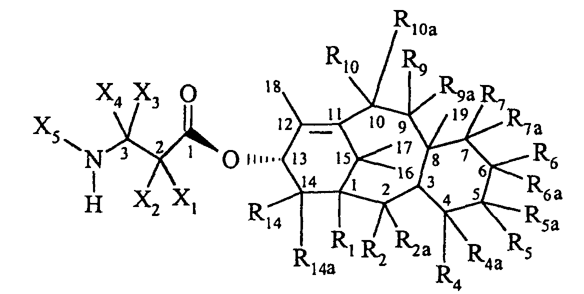 dans laquelle
X₁ est un groupe -OX₆, -SX₇ ou -NX₈X₉;
X₂ est un atome d'hydrogène, un groupe alkyle, alcényle, alcynyle, aryle ou hétéroaryle;
X₃ et X₄ sont indépendamment un atome d'hydrogène, un groupe alkyle, alcényle, alcynyle, aryle, hétéroaryle, acyle ou un groupe alkyle, alcényle, alcynyle, aryle ou hétéroaryle substitué par un hétéroatome, à condition, cependant, que X₃ et X₄ ne soient pas tous deux un groupe acyle;
X₅ est un groupe -COX₁₀, -COOX₁₀, -COSX₁₀, -CONX₉X₁₀ ou -SO₂X₁₁;
X₆ est un atome d'hydrogène, un groupe alkyle, alcényle, alcynyle, aryle, hétéroaryle ou un groupe protecteur du groupe hydroxy;
X₇ est un groupe alkyle, alcényle, alcynyle, aryle, hétéroaryle ou un groupe protecteur du groupe sulfhydryle;
X₈ est un atome d'hydrogène, un groupe alkyle, alcényle, alcynyle, aryle, hétéroaryle ou un groupe alkyle, alcényle, alcynyle, aryle ou hétéroaryle substitué par un hétéroatome;
X₉ est un groupe protecteur du groupe amino;
X₁₀ est un groupe alkyle, alcényle, alcynyle, aryle, hétéroaryle ou un groupe alkyle, alcényle, alcynyle, aryle ou hétéroaryle substitué par un hétéroatome;
X₁₁ est un groupe alkyle, alcényle, alcynyle, aryle, hétéroaryle, -OX₁₀ ou -NX₈X₁₄;
X₁₄ est un atome d'hydrogène, un groupe alkyle, alcényle, alcynyle, aryle ou hétéroaryle;
R₁₄ est un atome d'hydrogène, un groupe alkyle, alcényle, alcynyle, aryle ou hétéroaryle, un groupe hydroxy ou hydroxy protégé ou forme un carbonate avec le groupe R₁;
R₁₄ₐ est un atome d'hydrogène, un groupe alkyle, alcényle, alcynyle, aryle ou hétéroaryle;
R₁₀ est un atome d'hydrogène ou forme un groupe oxo avec le groupe R₁₀ₐ;
R₁₀ₐ est un atome d'hydrogène, un groupe -OCOR₂₉, hydroxy ou hydroxy protégé ou forme un groupe oxo avec le groupe R₁₀;
R₉ est un atome d'hydrogène ou forme un groupe oxo avec le groupe R₉ₐ;
R₉ₐ est un atome d'hydrogène, un groupe hydroxy, hydroxy protégé, acyloxy ou forme un groupe oxo avec le groupe R₉;
R₇ₐ est un atome d'hydrogène ou forme un groupe oxo avec le groupe R₇;
R₇ est un groupe hydroxy ou forme un groupe oxo avec le groupe R₇ₐ;
R₆ est un atome d'hydrogène, un groupe alkyle, alcényle, alcynyle, aryle ou hétéroaryle, un groupe hydroxy ou hydroxy protégé ou forme un groupe oxo avec le groupe R₆ₐ;
R₆ₐ est un atome d'hydrogène, un groupe alkyle, alcényle, alcynyle, aryle ou hétéroaryle, un groupe hydroxy, hydroxy protégé ou forme un groupe oxo avec le groupe R₆;
R₅ est un atome d'hydrogène ou forme un groupe oxo avec le groupe R₅ₐ;
R₅ₐ est un atome d'hydrogène, un groupe hydroxy, hydroxy protégé, acyloxy, il forme un groupe oxo avec le groupe R₅ ou il forme un cycle oxétane avec le groupe R₄ et les atomes de carbone auxquels ils sont liés;
R₄ est un atome d'hydrogène, il forme un groupe oxo avec le groupe R₄ₐ ou il forme un cycle oxétane avec le groupe R₅ₐ et les atomes de carbone auxquels ils sont liés;
R₄ₐ est un atome d'hydrogène, un groupe alkyle, alcényle, alcynyle, aryle, hétéroaryle, cyano, hydroxy, -COOR₃₀ ou forme avec le groupe R₄ un groupe oxo, oxirane ou méthylène;
R₂ est un atome d'hydrogène, un groupe hydroxy ou -OCOR₃₁;
R₂ₐ est un atome d'hydrogène ou forme un groupe oxo pris ensemble avec le groupe R₂;
R₁ est un atome d'hydrogène, un groupe hydroxy, hydroxy protégé;
R₂₉, R₃₀ et R₃₁ sont indépendamment un atome d'hydrogène, un groupe alkyle, alcényle, alcynyle, aryle monocyclique ou hétéroaryle monocyclique;
à condition cependant qu'au moins un des groupes X₃, X₄ et X₁₀ soit un groupe furyle ou thiényle; et que soit (i) R₁₀ₐ soit un atome d'hydrogène ou forme un groupe oxo avec le groupe R₁₀; soit (ii) R₉ₐ soit un atome d'hydrogène, un groupe hydroxy, hydroxy protégé ou acyloxy; soit (iii) R₇ forme un groupe oxo avec le groupe R₇ₐ; soit (iv) R₄ₐ soit un atome d'hydrogène, un groupe alkyle, alcényle, alcynyle, aryle, hétéroaryle, cyano, hydroxy, -OCOR₃₀, dans lequel R₃₀ est différent d'un groupe méthyle, ou forme avec le groupe R₄ un groupe oxo, oxirane ou méthylène; soit (v) R₂ soit un atome d'hydrogène, un groupe hydroxy ou -OCOR₃₁ dans lequel R₃₁ est différent d'un groupe phényle; soit (vi) R₁ soit différent d'un groupe hydroxy; soit (vi) R₁₄ soit différent d'un atome d'hydrogène.

2. Dérivé de taxane selon la revendication 1, dans lequel R₁₀ₐ est un atome d'hydrogène ou forme un groupe oxo avec le groupe R₁₀ ou R₉ₐ est un atome d'hydrogène, un groupe hydroxy, hydroxy protégé ou acyloxy.

3. Dérivé de taxane selon la revendication 1 ou la revendication 2, dans lequel R₄ₐ est un atome d'hydrogène, un groupe alkyle, alcényle, alcynyle, aryle, hétéroaryle, cyano, hydroxy, -OCOR₃₀, dans lequel R₃₀ est différent d'un groupe méthyle, ou forme avec le groupe R₄ un groupe oxo, oxirane ou méthylène ou R₂ est un atome d'hydrogène, un groupe hydroxy ou -OCOR₃₁, dans lequel R₃₁ est différent d'un groupe phényle.

4. Dérivé de taxane selon l'une quelconque des revendications 1 à 3, dans lequel X₃ ou X₄ est un groupe thiényle, X₅ est un groupe -COOX₁₀ et X₁₀ est différent d'un groupe phényle et alcoxy.

5. Dérivé de taxane selon l'une quelconque des revendications 1 à 4, dans lequel X₅ est un groupe -COX₁₀, X₁₀ est un groupe furyle ou thiényle et ni X₃ ni X₄ n'est un groupe phényle ou phényle substitué par un groupe para-nitro.

6. Dérivé de taxane selon l'une quelconque des revendications 1 à 5, dans lequel R₁ est différent d'un groupe hydroxy ou R₁₄ est différent d'un atome d'hydrogène.
